# EUROPEAN PATENT APPLICATION

(11) **EP 4 491 631 A1**
(43) Date of publication of application: **15.01.2025**
(21) Application number: 23185440.7
(22) Date of filing: 13.07.2023
(51) Int. Cl.: C07K 16/24, C07K 16/28

(54) **ANTI-PD-1 ANTIBODY BINDING DOMAIN AND IMMUNOCONJUGATE**

(71) Applicant: Anaveon AG, 4057 Basel (CH)
(72) Inventor: The designation of the inventor has not yet been filed
(74) Representative: Schulz Junghans Patentanwälte PartGmbB

(57) **Abstract**

The invention relates to anti-PD-1 antibody binding domains which do not disrupt signaling between PD1 and PD-L1, and immunoconjugates comprising same targeting an immunomodulatory domain to PD-1-expressing cells.

## Description

### Field

The present invention relates to an anti-PD-1 antibody binding domain compatible with checkpoint inhibitor agents configured to disrupt signaling between PD-1 and its ligands. The invention further relates to immunoconjugates comprising said anti-PD-1 binding domain, and methods of treating cancer patients with same, optionally in combination with PD-1 or PD-L1 antagonist antibodies.

### Background

The efficacy of anti-tumor activity of immunotherapeutic molecules, such as recombinant cytokines, can be improved by the inclusion of a domain targeting the molecule to where it is needed in the tumor or to the tumor microenvironment, for example. For example, IL-2 constructs having a tumour-specific antibody binding domain were shown to have a synergistic efficacy when used to treat cancer models in combination with a checkpoint inhibition antibody targeting PD-L1 (Klein C. 2017 Oncoimmunology **6**:e1277306). Alternatively, IL-2 molecules can be targeted to a specific immune cell with anti-tumour properties, for example by means of a targeting domain binding to CD8, PD-1 or other antigens expressed on the surface of CD8 T cells. Because these constructs present IL-2 and at the same time bind to a T cell surface, they are often called 'cis-signaling' IL-2 bispecifics or immunoconjugates. Such cis-signaling fusion proteins have been generated to create anti-PD-1/IL-2 bispecifics, (WO2018184964A1, Deak L. C. et al. 2022, Nature 610:161) and were shown to have superior efficacy to non-targeted IL-2 in mouse pre-clinical models.

In known anti-PD-1/IL-2 constructs, the targeting arm consists of blocking anti-PD-1 antibodies (blocking PD-1/PD-1L interactions). Such a targeting arm has the potential advantage of relieving PD-L1 induced T cell suppression while also delivering the proliferative IL-2 signal to the same effector cells. There is, however, an inherent pharmacological difficulty in administering a blocking therapeutic (requiring dosing providing full coverage of the target) and an agonistic therapeutic (requiring dosing amount and frequency sufficient for a safe and agonistic effect). In the specific case of PD-1 and IL-2 therapeutics, anti-PD-1 antibodies are administered in mg/kg doses to ensure full target coverage, but cytokines such as IL-2 are administered in microgram/kg doses to avoid overstimulation and on-target toxicity. The discrepancy has been addressed for example by mutating the IL-2 portion of the molecule to reduce affinity to its receptor and thereby allow higher doses of the bispecific. Mutations to the IL-2 structure, however, may result in anti-drug antibodies which can render the therapy ineffective or toxic. Furthermore, in patients receiving PD-1 check point inhibitor (CPi) therapy, the epitope targeted by the PD-1/IL-2 fusion protein is already occupied by the CPi, preventing binding of the fusion protein to the targeted cells, making the drug incompatible as an adjunct to standard checkpoint inhibition treatment regimes.

Based on the above-mentioned state of the art, the objective of the present invention is to provide a PD-1-targeting domain compatible with existing therapeutic agents which disrupt signaling between PD-1 and its ligands . This objective is attained by the subject-matter of the independent claims of the present specification, with further advantageous embodiments described in the dependent claims, examples, figures and general description of this specification.

### Summary of the Invention

The inventors have developed PD-1 targeted antibody binding domains, which do not inhibit the binding or antagonist action of conventional PD-1, or PD-L1 targeted checkpoint inhibitor antibodies. The anti-PD-1 binding domains of non-blocking antibodies are used as a targeting moiety in immunoconjugates facilitating targeted delivery of an immune modulating polypeptide, delivering IL-2 signal to the appropriate exhausted CD8 T cells. This results in an immunoconjugate compound that effectively delivers a payload to PD-1 expressing cells, while allowing modulated dosing of potent active agents to achieve safe and efficacious treatment of cancer.

A first aspect of the invention relates to immunoglobulin (Ig) variable domains capable of binding to PD-1 in the presence of an at least equimolar amount of a PD-1-specific checkpoint inhibitor antibody. Interaction of an antibody, or immunoconjugate comprising the Ig variable domain according to the invention with PD-1 is compatible with simultaneous binding of an anti-PD-1 checkpoint inhibitor antibody, preserving their immune-stimulatory effect. In certain embodiments, the binding of an antibody characterized by the immunoglobulin variable domain according to the invention is no more than 20% reduced in the presence of a 100-fold molar excess of an anti-PD-1 checkpoint inhibitor antibody (for example pembrolizumab, or nivolumab). In particular embodiments, such an antibody or immunoconjugate comprising the Ig variable domain according to the invention is characterized by a high affinity for PD-1 of as characterized by an affinity constant (K_{D}) of 1.0x10⁻⁹ mol/L or lower.

The non-blocking Ig variable domain specific for PD-1 is comprised of both an antibody heavy chain variable domain polypeptide (PD1-VH), and an antibody light chain variable domain polypeptide (PD1-VL). The PD1-VH and PD1-VL associate as a heterodimer, providing a functional antibody antigen-binding domain.

In some embodiments, the sequence of the PD1-VH comprises the following heavy chain complementary determining regions (HCDR) 1-3: a HCDR1 having the sequence SEQ ID NO 118, an HCDR2 having the sequence SEQ ID NO 119, and an HCDR3 having the sequence SEQ ID NO 120, while the PD1-VL comprises the light chain complementary determining regions (LCDR): LCDR1 having the sequence SEQ ID NO 121, LCDR2 having the sequence SEQ ID NO 122, and LCDR3 with the sequence SEQ ID NO 123.

A next aspect of the invention relates to immunoconjugates comprising the non-blocking anti-PD-1 antigen binding variable domain as specified in the aspect of the invention described above. The immunoconjugate additionally comprises an immune-active polypeptide component delivering a signaling effect to a target other than PD-1, such as an interleukin, or an interleukin receptor, particularly a target that is also expressed by PD-1+ cells such as T cells or natural killer cells.

In some embodiments, the immunoconjugate comprises a non-blocking PD-1 antibody variable domain, and an interleukin joined to an Ig antibody binding domain specific for said interleukin, particularly a non-blocking PD-1 specific domain together with an IL-2 polypeptide linked to an anti-IL-2 antibody binding domain as illustrated in the examples in a variety of functional formats.

Further aspects of the invention relate to isolated nucleic acid sequences, expression vectors or cells expressing or encoding anti-PD-1 antibody variable domains and/or immunoconjugates as described herein.

The invention further relates to pharmaceutical compositions comprising immunoconjugates according to the invention, and their use in treating cancer, in addition to methods of treating cancer patients by administering an effective amount of immunoconjugates according to the invention.

### Terms and definitions

The term *PD-1* in the context of the present specification relates to the human PD-1 protein, encoded by the *PDCD1* gene, also sometimes referred to as CD279 (Uniprot Q15116).

The term *PD-L1* in the context of the present specification relates to the human PD-L1 protein, encoded by the gene *CD274*, also sometimes referred to as CD274 (Uniprot Q9NZQ7).

The term *IL-2* in the context of the present specification relates to human IL-2, and functional variants thereof, such as the wildtype human amino acid sequence (Uniprot P60568), and variant proteins listed in Table 2.

The term *IL2CP, or IL2-CP* in the context of the present specification relates to a circularly permuted IL-2 polypeptide is created by "opening" the IL-2 polypeptide sequence, to create a new N' terminus and C' terminus, and fusing natural N' and C' terminal amino acid residues. This generates a reordered IL2CP polypeptide retaining important tertiary structures of the cytokine that allow signaling through dedicated receptors.

The term *dimeric IL-2 receptor* in the context of the present specification relates to the heterodimer receptor comprising the two IL-2 receptor chains CD122 and CD132.

Any patent document cited herein shall be deemed incorporated by reference herein in its entirety.

In the context of the present specification, the term *dimer* refers to a unit consisting of two subunits. In the context of the present specification, the term *heterodimer* refers to a dimer comprised of two subunits that are not identical.

The terms *heterotetramer Ig, heterotetrameric Ig,* refer to recombinant immunoglobulin-like molecules comprising a first heavy chain and light chain pair characterized by a first antibody binding domain specific for a first antigen, associated together with a second heavy chain and light chain pair characterized by a second antibody binding domain specific for a second antigen. This is also sometimes referred to as a bispecific antibody, and includes such formats as a heterotetrameric kappa/lambda format, and the Crossmab format (WO2009080253).

In the context of the present specification, the term *amino acid linker* or *peptide linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids.

There is no constraint on the amino acid composition of the linker. In certain embodiments, the linker consists of amino acids selected from the group of G S, A and D. An important characteristic of the conjugate peptide linkers as specified herein are low immunogenicity, and a peptide length that allows the domains which are joined by the linker to interact to form a functional entity such as the immunoconjugates as disclosed herein. In particular desirable embodiments of the domain peptide linkers specified above, the sequences are primarily made up of stretches of small, polar amino acids such as glycine (G) and serine (S).

In certain embodiments peptide linker is at least (≥) 15 amino acids in length, particularly 15 to 30 amino acids in length wherein the amino acids are selected from G S, A and D.

A non-limiting example of an amino acid linker is a monomer or di-, tri- or tetramer of a peptide motif composed of three or four glycine and one serine.

Any embodiments relating peptide linkers as disclosed herein, encompass structures in which amino acids with similar characteristics are exchanged, for example, the amino acids V, L, I, P, S, C, or M may replace G, S, or S, and D may be replaced by E.

Particular non-limiting examples of linkers are provided by SEQ ID NO 014, 015, 016, 017, 018, 019, 020, 021, 022, 023, 024 and 025.

### (Cancer) Immunotherapy

In the context of the present specification, the term *PD-1 antagonist antibody* is meant to encompass an agent, particularly an antibody (or antibody-like molecule) capable of disrupting the signal cascade leading to T cell inhibition after T cell activation as part of what is known in the art the immune checkpoint mechanism. Non-limiting examples include antibodies to PD-1 (Uniprot Q15116) or to PD-L1 (Uniprot Q9NZQ7), such as exemplified by the clinically available antibody drugs nivolumab (Bristol-Myers Squibb; CAS No 946414-94-4), pembrolizumab (Merck Inc.; CAS No. 1374853-91-4), dostarlimab (Tesaro; CAS No 2022215-59-2), sintilimab (Eli Lilly, InnoVent Biologics; CAS No 2072873-06-2), tislelizumab (BeiGene; CAS No 1858168-59-8), cemiplimab (CAS No 1801342-60-8), cetrelimab (Cas No 2050478-92-5), sasanlimab (Cas No 2206792-50-7).

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

The term *cancer* as used in the context of the present specification relates to malignant neoplastic disease; the terms "cancer" and "malignant neoplastic disease" are used synonymously herein. They specifically include carcinoma (epithelial derived cancer), sarcoma (connective tissue derived cancer), lymphoma and leukemia, germ-cell derived tumors and blastomas. Particular alternatives of any of the aspects and embodiments disclosed herein are directed at the use of the compounds and compositions of the invention in treatment of solid tumors. Other alternatives of any of the aspects and embodiments disclosed herein are directed at the use of the combinations of the invention in treatment of liquid cancers such as myelogenous or granulocytic leukemia, particularly AML, lymphatic, lymphocytic, or lymphoblastic leukemia and lymphoma, polycythemia vera or erythema.

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

### Non-blocking immunoglobulin antigen binding domains specific for PD-1

A first aspect of the invention is an immunoglobulin (Ig) variable domain capable of binding to PD-1 in the presence of an at least equimolar amount of a PD-1-specific checkpoint inhibitor antibody. Interaction of an antibody, or immunoconjugate comprising the Ig variable domain according to the invention with PD-1 is compatible with co-administration of anti-PD-1 checkpoint inhibitor antibodies in vitro and in vivo, because it does not significantly inhibit their immune-stimulatory function as shown for example, in the in vitro assay summarised in table 7.

Non-blocking Ig variable domains capable of binding PD-1 according to the invention comprises both an antibody heavy chain variable domain polypeptide (PD1-VH), and an antibody light chain variable domain polypeptide (PD1-VL), associated as a heterodimer to form a functional antibody antigen-binding domain.

In certain embodiments, the non-blocking feature of the Ig variable domain binding to PD-1 in the presence of a PD-1 checkpoint inhibitor antibody, is characterized by assessment of the binding of an antibody having a two heavy and two light chains forming two antibody PD-1 binding domains according to the invention. According to these embodiments, the binding of such an antibody to PD-1 expressed on the surface of a cell, is no more than 20% reduced in the presence of a 100-fold molar excess of a PD-1 agonist selected from pembrolizumab, or nivolumab.

The PD-1 checkpoint inhibitor non-blocking feature of a PD-1-binding Ig variable domain according to the invention can by assessing binding to PD-1 expressed by a mammalian cell (e.g. Jurkat cells expressing PD-1 as demonstrated in Example 3). In brief, PD-1 expressing cells are incubated for 30 minutes at 4°C with a serial dilution of pembrolizumab or nivolumab. Without washing the cells, antibodies characterized by an immunoglobulin variable domain according to the invention are added at a fixed concentration of 100 nMol/L, labelled with a detectable such as biotin, or a fluorochrome. Bound antibodies are then detected by means of flow cytometry. To determine the percent of binding inhibition by pembrolizumab, or nivolumab, the mean fluorescence intensity (MFI) of bound antibody is compared that of control samples without competitor to identify the percent of binding inhibition at the desired level of molar excess of checkpoint inhibitor.

In some embodiments of the Ig variable domain capable of binding PD-1 according to the invention, it binds to PD-1 without significantly inhibiting the interaction of PD-1 with pembrolizumab. In other embodiments, it binds to PD-1 without significantly inhibiting the interaction of PD-1 with nivolumab.

An Ig variable domain binding PD-1 according to the invention, or an antibody characterized by said binding domain, binds specifically to the human protein PD-1 expressed on the surface of a cell. Preferably, the antibody is also highly specific for primate PD-1, to facilitate pre-clinical testing. In particular embodiments, the affinity constant (K_{D}) for PD-1 of a classic antibody structure characterized by Ig variable domains according to the invention (i.e. two heavy chains and lights chains association to provide two of said immunoglobulin variable domains) is 1.0x10⁻⁹ mol/L or lower as measured using surface plasmon resonance as described in the section *Binding; Binders Ligands Antibodies* above.

In some embodiments of the immunoglobulin variable domain capable of binding to PD-1 according to the invention, it is characterized by a PD1-VH which comprises a heavy chain CDR (HCDR) 1 (HCDR1) having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120). The PD1-VL comprises an LCDR1 having the sequence SGX¹SSNIGS(XX)²VF (where X¹ is N, S, Q, or A, and (XX)² is NS, QS, SS, or NA) (SEQ ID NO 121), an LCDR2 having the sequence SNNQRPS (SEQ ID NO 122), and an LCDR3 having the sequence AAWDDSLSIWVF (SEQ ID NO 123).

In particular embodiments, the LCDR1 is characterized by SEQ ID NO 121 where X¹ is S, Q, or A and (XX)² is QS, SS, or NA. These CDR correspond to derivatives of the antibody clone 21A08 with favorable non-blocking characteristics, and high affinity for PD-1 (both human PD-1) and macaque). The optional modifications at site X¹ remove NS deamidation site of this clone without compromising PD-1 binding, as all alternatives generated preserved hPD-1 and cPD-1 binding (see Table 15). Alternatively, or in addition, modifications at (XX)² may remove an N-glycosylation site present in the LCDR1 of 21A08. All alternatives tested preserved favourable binding to PD-1 compared to the parent sequence (see Table 14).

In certain embodiments of the Ig variable domain according to preceding paragraph, the binding affinity K_{D} for PD-1 of an antibody characterized by said Ig variable domain is in the range of 1.0×10⁻⁹ to 1.5×10⁻¹¹ mol/L as measured by SPR. In particular embodiments, the K_{D} is in the range of 1.0×10⁻¹⁰ to 1.5×10⁻¹¹ mol/L. In more particular embodiments, the K_{D} is in the range of 5.0×10⁻¹⁰ to 1.5×10⁻¹¹ mol. This high PD-1 affinity distinguishes the binding domain of the invention from known PD-1 non-blocking clones such as XVT458, for which the K_{D} was in the range of 1.28 × 10⁻⁸ nM (Table 10) or 1.6 × 10⁻⁸ nM (Table 8).

In certain embodiments of variable domains according to invention relating to the clone generated herein named 21A08P1, the PD1-VL comprises an LCDR1 having the sequence SGASSNIGSQSVF (SEQ ID NO 124). In particular embodiments of the 21A08P1 Ig variable domain, the PD1-VH comprises, or consists of a polypeptide at least (≥) 95%, ≥ 98%, ≥ 99% similar to SEQ ID NO 085, and the PD1-VL comprises, or consists of a polypeptide ≥ 95%, ≥98 %, ≥ 99% similar to SEQ ID NO 086. In more particular embodiments, the PD1-VH comprises a polypeptide having the sequence SEQ ID NO 085 and the PD1-VL comprises a polypeptide having the sequence SEQ ID NO 086. In still more particular embodiments, the PD1-VH consists of the polypeptide SEQ ID NO 085 and the PD1-VL consists of the polypeptide SEQ ID NO 086.

Further embodiments of an 21A08 Ig variable domain according to invention relate to the clone herein named 21A08P2, where the PD1-VL comprises an LCDR1 having the sequence SGASSNIGS**SS**VF (SEQ ID NO 125). In particular embodiments of the 21A08P2 Ig variable domain, the PD1-VH comprises, or consists of a polypeptide at least (≥) 95%, ≥ 98%, ≥ 99% similar to SEQ ID NO 085, and the PD1-VL comprises, or consists of a polypeptide ≥ 95%, ≥98 %, ≥ 99% similar to SEQ ID NO 087. In more particular embodiments, the PD1-VH comprises a polypeptide having the sequence SEQ ID NO 085 and the PD1-VL comprises a polypeptide having the sequence SEQ ID NO 087. In still more particular embodiments, the PD1-VH consists of the polypeptide SEQ ID NO 085 and the PD1-VL consists of the polypeptide SEQ ID NO 087.

Further embodiments of an 21A08 Ig variable domain according to invention relate to the clone derived herein named 21A08P3, where the PD1-VL comprises an LCDR1 having the sequence SGASSNIGS**NA**VF (SEQ ID NO 126). In particular embodiments of the variable domain, the PD1-VH comprises, or consists of a polypeptide at least (≥) 95%, ≥ 98%, ≥ 99% similar to SEQ ID NO 085, and the PD1-VL comprises, or consists of a polypeptide ≥ 95%, ≥98 %, ≥ 99% similar to SEQ ID NO 091. In more particular embodiments, the PD1-VH comprises a polypeptide having the sequence SEQ ID NO 085 and the PD1-VL comprises a polypeptide having the sequence SEQ ID NO 091. In still more particular embodiments, the PD1-VH consists of the polypeptide SEQ ID NO 085 and the PD1-VL consists of the polypeptide SEQ ID NO 091.

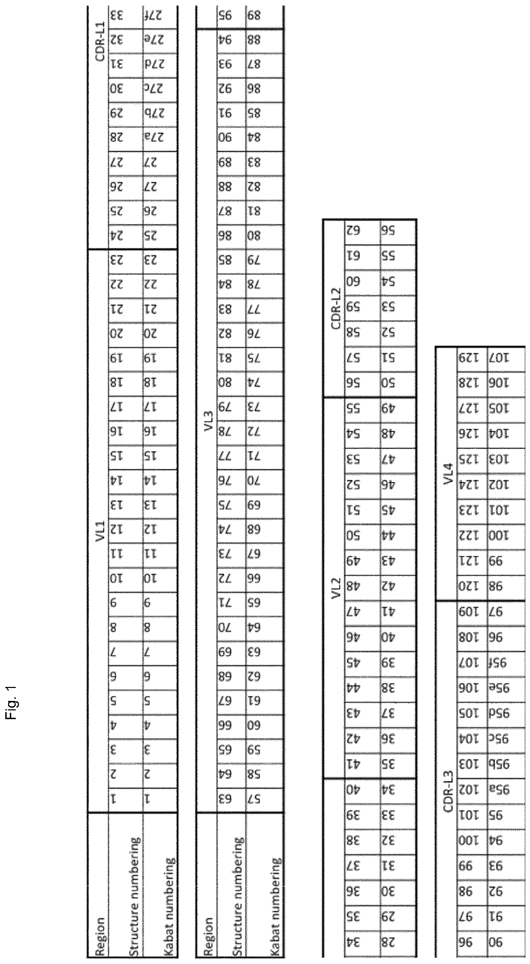

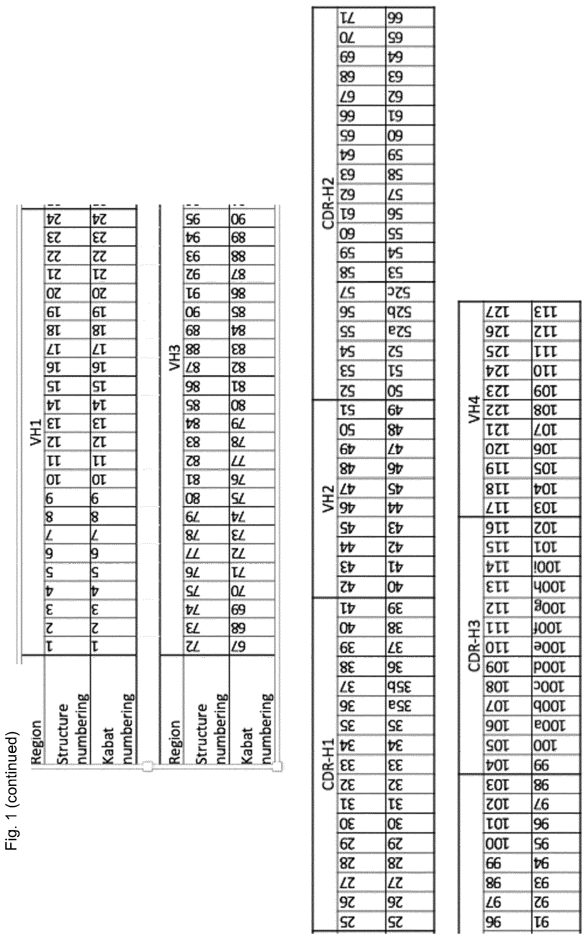

Another aspect of the invention relates to Ig variable domains capable of binding to PD-1 where the PD1-VH comprises, or consists of a polypeptide ≥ 95%, ≥ 98%, ≥ 99% similar to SEQ ID NO 071 and the PD1-VL comprises, or consists of a polypeptide ≥ 95%, ≥ 98%, ≥ 99% similar to SEQ ID NO 072. In particular embodiments, the polypeptide ≥ 95% to SEQ ID NO 071 has a conserved HCDR1, an HCDR2, and an HCDR3 identical to the CDRs annotated in SEQ ID NO 071 while the LCDR1, LCDR2, and LCDR3 of the polypeptide ≥ 95% to SEQ ID NO 072 are identical to those annotated in SEQ ID NO 072. Such similar PD1-VH and PD1-VL preserve the binding epitope of the clone 21A08 having high specificity for PD-1 and not blocking PD-1 checkpoint inhibitor antibody binding, by not introducing any amino acid substitutions into CDR regions (Fig 1).

### Non-blocking immunoconjugates binding PD-1

An immunoconjugate is an antibody, or antibody fragment or antibody-like molecule such as an ScFV conjugated or joined to an additional functional moiety. A further aspect of the invention is an immunoconjugate comprising a targeting moiety corresponding to any one of the immunoglobulin variable domains described in the previous section *Non-blocking immunoglobulin antigen binding domain specific for PD-1.* The targeting moiety is conjugated to an immune modulating domain capable of delivering a signaling effect to a PD-1-expressing cell. Due to the unique PD-1 epitopes targeted by binding domains according to the invention, co-administration of an immunoconjugate (or delivery directly prior to, or subsequent to) to a subject with a checkpoint inhibitor antibody such as an anti-PD-1 antagonist antibody, does not significantly inhibit the immune-stimulatory function of the anti-PD-1 antagonist antibody. In particular embodiments, the immunoconjugate is compatible with co-administration of (i.e. does not interfere with the immunostimulatory properties of) an anti-PD-1 antagonist antibody selected from the list consisting of nivolumab, pembrolizumab, dostarlimab, sintilimab, tislelizumab, cemiplimab, cetrelimab, sasanlimab. In more particular embodiments, the immunoconjugate is compatible for co-administration within a medically relevant window of nivolumab or pembrolizumab.

Further embodiments of the invention relate to immunoconjugates comprising an additional immune-active polypeptide ligand capable of binding to a cell surface molecule expressed by immune cells, in addition to the Ig variable domain binding PD-1. In particular embodiments, the immune-active polypeptide ligand binds specifically to a cell surface molecule expressed by T cells or natural killer cells. These cells express PD-1 and are desirable targets for PD-1-targeting immune modulating activating or inhibiting agents such as IL-2 due to their known anti-tumour effector properties, however other cells can express PD-1 as well.

In particular embodiments, the immune-active polypeptide ligand binds specifically to a cell surface molecule expressed by CD8 T cells, such as inhibitory co-receptors such as LAG-3 or CTLA-4, cell surface receptors, such as the T cell receptor components, or interleukin receptors, or adhesion molecules such as integrins.

Further embodiments of the immunoconjugate according to the invention comprise an immune-active polypeptide ligand comprising an interleukin. In certain embodiments, the immune-active polypeptide ligand portion of the immunoconjugate consists of an interleukin. In particular embodiments of the immunoconjugate according to the invention, the immune-active polypeptide ligand portion is, or comprises an interleukin 2 (IL-2) polypeptide.

In some embodiments of the immunoconjugate according to the invention, the immune-active polypeptide ligand comprises, or consists of an immunoglobulin variable domain reactive to, i.e binding specifically to, an interleukin. In different embodiments of the immunoconjugate according to the invention, the immune-active polypeptide ligand comprises, or consists of an immunoglobulin variable domain reactive to an interleukin receptor.

Certain embodiments of the immunoconjugate according to the invention relate to immune-active polypeptide ligands comprising both an interleukin, and an immunoglobulin variable domain reactive to said interleukin. Optionally, a peptide linker joins the interleukin to either the light chain, or the heavy chain of the immunoglobulin variable domain reactive to said interleukin. Such antibody domain, interleukin fusions, can orient the interleukin to enhance a certain signaling function, for example deliver biased signaling of IL-2 to IL-2 receptors on effector CD8+ T cell expressing PD-1, with limited bystander activation of T regulatory T cells.

### IL-2 polypeptides presented in the context an anti-IL-2 antibody binding domain

Some embodiments of the immunoconjugate according to the invention comprise a PD-1 binding domain, joined to an immune-active polypeptide ligands comprising both an anti-IL-2 binding domain (a heavy chain polypeptide and a light chain polypeptide derived from an anti-IL-2 antibody capable of binding specifically to the human IL-2 protein) and an IL-2 polypeptide covalently linked to amino acid residues of the anti-IL-2 binding domain. This linkage, optionally in the form of on one, or two peptide linkers, provides a single contiguous recombinant polypeptide, enhancing biased signaling to the dimeric IL-2 receptor compared to an unbound IL-2 polypeptide such as Proleukin.

Embodiments of an IL-2 polypeptide portion of the immunoconjugate according to the invention include human IL-2, artificial IL-2 variant polypeptides such as Proleukin, or IL-2 muteins as provided in Table 2. The IL-2 polypeptide according to the invention lacks the signal peptide of M1 to S21 of native IL-2 SEQ ID NO 005.

### Circularly permuted IL-2 polypeptides

In particular embodiments of the immunoconjugate according to the invention, the IL-2 polypeptide is a circularly permuted IL-2 (IL2CP), having functional domains reordered relative to their domain position in the wildtype sequence of SEQ ID NO 005, such as the examples provided in in Table 3. Particular embodiments of an IL-2 polypeptide starting sequence to which circular permutation can be applied are WT SEQ ID NO 005, SEQ ID NO 006 or the muteins listed in the Table 2.

In particular embodiments of the immunoconjugate according the invention, it comprises an IL2CP embedded within the IL-2-VH, or the IL-2-VL, flanked, or joined, at each end by a short peptide linker. In more particular embodiments, both the first and second peptide linkers are 1-20 amino acids in length. In still more particular embodiments both the first and second peptide linkers are between 2 and 7 amino acids in length. In further particular embodiments of the immunoconjugate according to the invention, any peptide linker flanking the IL2CP is comprised of glycine (G), or G and serine (S) residues. In more particular embodiments, the peptide linker joining the C- or N-terminal residue of the IL2 polypeptide to amino acids of the IL-2VH or IL-VL has a sequence selected from the sequences assigned as SEQ ID NO 014-024.

### Anti-IL-2 binding domains

In certain embodiments of the immunoconjugate according to the invention, the immune active polypeptide ligand comprises a polypeptide having the sequence of SEQ ID NO 167 (IL2 embedded into the LC variable domain used in QTY065), and a polypeptide having the sequence of SEQ ID NO 043 (corresponding variable domain in the HC used in QTY065).

In certain embodiments of the immunoconjugate according to the invention, the anti-IL-2 antigen binding domain comprises, or consists of, an IL2-VH having sequence SEQ ID NO 044 (Antibody B VH), associated with an IL2-VL selected from SEQ ID NO 032 to 036. These embodiments relate to IL2CP embedded into LCDR1 of Antibody B, EPK959, GYG794, DRO069, or ECV200, and BFC885. These also showed little binding to CD25 as measured by SPR (Table 5). These validated alternatives of IL2CP composition and linkage embedding, deliver biased signaling to the dimeric IL-2 receptor according to the invention.

### Fc portions

In certain embodiments of the immunoconjugate according to the invention, the immunoconjugate comprises an Fc portion, conferring extended half-life in vivo. In particular embodiments, the Ig Fc is an IgG Fc portion.

In certain embodiments of the immunoconjugate, the IgG Fc portion is characterized by the presence of one or more modifications to constant regions of the heavy chains to enhance correct heavy chain pairing. In particular embodiments, the modifications are selected from the following knob and hole paired mutations to enhance heavy chain pairing:
- Knob: S354C, T366W and Hole: Y349C, T366S, L368A, Y407V;
- Knob: T366Y, and Hole Y407T;
- Knob: Y349C T366W, and Hole: S354C, T366S, L368A, Y407V;
- Knob: T366W, and Hole: Y407A, T366S, L368A.

In certain embodiments of the immunoconjugate according to the invention, the IgG Fc portion is characterized by the presence of one or more modifications to constant regions of the heavy chains to reduce effector function of the Fc portion. In particular embodiments, said modification, or modifications are selected from L234A, L235A (LALA), L234A, L235A, P329G (LALA-PG), L234A, L235A, P329A (LALA-PA), N297A, N297Q, N297G and D265A, N297G (DANG). In particular embodiments, the immunoconjugate comprises P329A (LALA-PA).

### Immunoconjugate formats

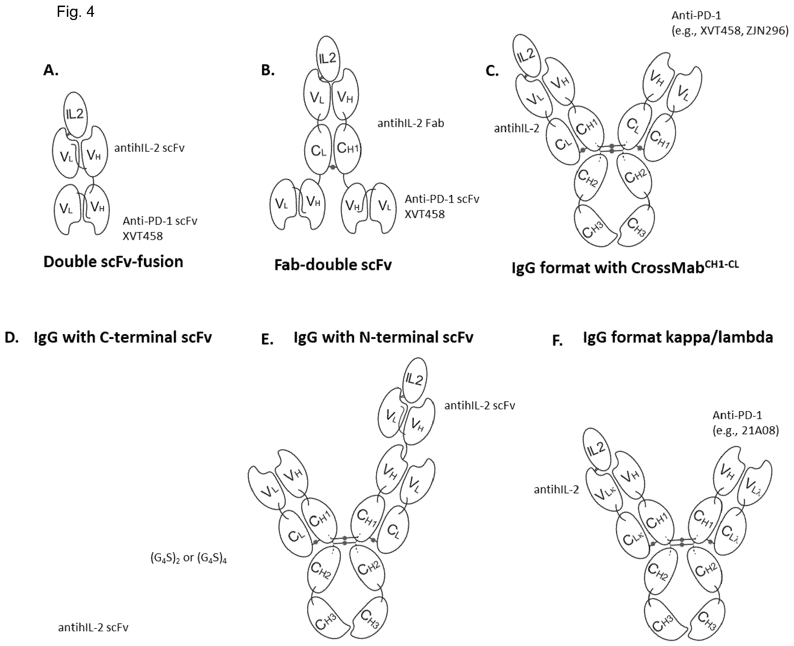

In certain embodiments of the immunoconjugate according to the invention, the immunoconjugate is a heterotetrameric IgG consisting of a first heterodimer comprising the non-blocking anti-PD-1 antibody binding domain, and a second heterodimer comprising the anti-IL2 binding domain. This format is illustrated in Figure 4, and includes such formats as a heterotetrameric kappa/lambda IgG format, and the Crossmab format (Fig. 4C, WO2009080253).

In particular embodiments of immunoconjugate according to invention, the immunoconjugate is a heterotetrameric kappa/lambda IgG. In particular embodiments, the antibody heavy and light chain heterodimer forming the anti-PD-1 Ig variable domain are characterized by a lambda light chain, for example those of the 21A08 clone family described above, paired with a heavy chain and kappa light chain forming an antibody binding domain specific to a second target, such as an interleukin, or cell surface molecule of a T cell or NK cell.

In particular embodiments, the immunoconjugate is a heterotetrameric IgG comprising a first antibody heavy and light chain heterodimer comprising the immunoglobulin variable domain capable of binding PD-1 characterized in that the light chain is a lambda light chain. Additionally, the immune-active polypeptide ligand portion of the immunoconjugate comprises, or consists of a second antibody heavy and light chain heterodimer comprising an immunoglobulin variable domain reactive to a cell surface molecule expressed by immune cells, characterized in that the light chain is a kappa light chain.

In further embodiments of the immunoconjugate according to the invention, the immunoconjugate is an immunoglobulin single chain variable fragment (scFv) format. As illustrated in Fig. 4, this is an antibody where both arms are specific for PD-1, further comprising a linked ScFv specific for a different antigen linked to the N-terminus of the PD-1 antibody heavy or light chain. Such an immunoconjugate has:
An anti-PD1 antibody comprising a first and a second antibody heavy chain and light chain heterodimer each comprising the anti-PD-1 immunoglobulin variable domain as specified in the section *Non-blocking immunoglobulin antigen binding domains specific for PD-1;* and
An interleukin, and an immunoglobulin scFv domain reactive to said interleukin. The immunoglobulin scFv domain is linked via a peptide linker to the N-terminal or the C-terminal residues of the heavy chain, or the light chain of the anti-PD-1 antibody.

In particular embodiments, the C-terminal residue of scFv domain reactive to said interleukin is linked via a peptide linker to the N-terminal residues of the heavy chain, or the light chain of the anti-PD1 antibody.

In particular embodiments of the immunoconjugate according to the invention, the immune active polypeptide ligand comprises a polypeptide having the sequence of SEQ ID NO 167 and a polypeptide having the sequence of SEQ ID NO 043.

In particular embodiments relating to one of the preferred formats assigned NZA596, the immunoconjugate comprises, or consists of the polypeptides having the sequences SEQ ID NO 111, SEQ ID NO 095, SEQ ID NO 112, and SEQ ID NO 052.

In particular embodiments relating to one of the preferred formats assigned XWY176, the immunoconjugate comprises, or consists of the polypeptides having the sequences SEQ ID NO 094, SEQ ID NO 095, SEQ ID NO 100, and SEQ ID NO 052;

In particular embodiments relating to one of the preferred formats assigned GQM289, the immunoconjugate comprises, or consists of the polypeptides having the sequences SEQ ID NO 097, SEQ ID NO 098, SEQ ID NO 100, and SEQ ID NO 052.

In particular embodiments relating to one of the preferred formats assigned LTJ498, the immunoconjugate comprises, or consists of the polypeptides having the sequences SEQ ID NO 094, SEQ ID NO 095, and SEQ ID NO 96.

In particular embodiments relating to one of the preferred formats assigned JLI141, the immunoconjugate comprises, or consists of the polypeptides having the sequences SEQ ID NO 097, SEQ ID NO 098, and SEQ ID NO 99.

### Vectors, cells, expressing domains or immunoconjugates according to the invention

Another aspect of the invention relates to an isolated nucleic acid encoding the immunoconjugate according to any one of the embodiments of the immunoconjugate aspect of the invention. Another aspect of the invention relates to an expression vector comprising said isolated nucleic acid. The invention further encompasses a host cell comprising said nucleic acid or said expression vector. In particular embodiments, the isolated nucleic acid is comprised in a mammalian expression vector under control of a promoter operable in a mammalian cell.

### Medical treatment and Medical use

Another aspect of the invention relates to a combination medicament comprising
i) an immunoconjugate as specified in any one of the aspects or embodiments of the invention herein, and
ii) an anti-PD-1 antagonist antibody.

In particular embodiments the anti-PD1 antagonist antibody is selected from the list consisting of nivolumab, pembrolizumab, dostarlimab, sintilimab, tislelizumab, cemiplimab, cetrelimab, sasanlimab. In more particular embodiments, the antibody is nivolumab or pembrolizumab.

Another aspect of the invention, is an immunoconjugate comprising a non-blocking anti-PD-1 binding domain according to the invention, for use in a patient who is receiving an anti-PD1 antagonist antibody selected from the list consisting of nivolumab, pembrolizumab, dostarlimab, sintilimab, tislelizumab, cemiplimab, cetrelimab, sasanlimab. In more particular embodiments, the antibody is nivolumab or pembrolizumab.

Another aspect of the invention is an anti-PD1 antagonist antibody selected from the list consisting of nivolumab, pembrolizumab, dostarlimab, sintilimab, tislelizumab, cemiplimab, cetrelimab, sasanlimab, for use in a patient receiving administration of an immunoconjugate according to the invention. In certain embodiments for use in a patient who has been administered the immunoconjugate according to the invention with the previous month.

The invention further encompasses nivolumab for use in a patient who is receiving administration of an immunoconjugate according to the invention. The invention further encompasses pembrolizumab for use in a patient who is receiving administration of an immunoconjugate according to the invention.

Similarly, the invention encompasses methods of treatment of a patient having been diagnosed with a cancer. This method entails administering to the patient an effective amount of immunoconjugate as identified herein, its pharmaceutically acceptable salt, as specified in detail herein, optionally in combination with CPi antibody.

In particular embodiments, the same subject is co-administered, within a medically relevant window, anti-PD1 antagonist antibody is selected from the list consisting of nivolumab, pembrolizumab, dostarlimab, sintilimab, tislelizumab, cemiplimab, cetrelimab, sasanlimab. In more particular embodiments, the antibody is nivolumab or pembrolizumab.

### Pharmaceutical Compositions, Administration/Dosage Forms and Salts

According to one aspect of the compound according to the invention, the immunoconjugate according to the invention is provided as a pharmaceutical composition, pharmaceutical administration form, or pharmaceutical dosage form, said pharmaceutical composition, pharmaceutical administration form, or pharmaceutical dosage form comprising the immunoconjugate of the present invention and at least one pharmaceutically acceptable carrier, diluent or excipient.

In certain embodiments of the invention, the immunoconjugate of the present invention is typically formulated into pharmaceutical dosage forms to provide an easily controllable dosage of the drug and to give the patient an elegant and easily handled product.

The invention further encompasses a pharmaceutical composition comprising an immunoconjugate of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of an immunoconjugate as specified in detail above, for use in a method of manufacture of a medicament for the treatment or prevention of a cancer.

Wherever alternatives for single separable features such as, for example, an isotype protein or coding sequence, or cancer are laid out herein as "embodiments", it is to be understood that such alternatives may be combined freely to form discrete embodiments of the invention disclosed herein. Thus, any of the alternative embodiments for an immunoconjugate may be combined with any of the alternative embodiments of a CPi compound and these combinations may be combined with any medical indication or diagnostic method mentioned herein.

The invention is further illustrated by the following examples and figures, from which further embodiments and advantages can be drawn. These examples are meant to illustrate the invention but not to limit its scope.

### Description of the Figures

- Fig. 1: shows labelling of antibody residues according to accepted formats with standard numbering and Kabat numbering systems.
- Fig. 2: shows how IL2-CP formats can be varied to suit embedding in various variable domain sites. (Left) a figure of the IL-2 with the correct orientation for binding to Antibody A or B (Middle) a figure of LCDR1 embedded CDAB IL-2CP in Antibody A, B or C (right) HCDR3 embedded BCDA IL-2CP.
- Fig. 3: SPR sensograms (Response Units, RU over Time in seconds) of PD-1 pre-mixed with antibodies or Buffer run as analyte on immobilized PD-L1.
- Fig. 4: shows proof of principal formats with various PD-1 binding moiety arrangement, including A. a double scFv-fusion, B. a Fab-double scFC, C. a heterotetrameric IgG (IgG CrossMab), D-E. two bivalent PD-1 IgG antibodies, with two linkage positions of an anti-hIL-2 ScFv F. a heterotetrameric IgG (kappa/lambda).
- Fig. 5: shows cell proliferation analyzed by flow cytometry, detecting Ki67+ cells in CD8 T cells, NK cells and Treg cells. Bispecific compounds administered i.v. at 0.2mg/kg to wt C57BL/6 mice. Blood samples were taken before the injection and on days 3 and 6 after compound administration.

### Examples

### Example 1: Design and production of anti-IL-2 antibody IL-2 cytokine fusion proteins

Antibodies binding to IL-2 were derived, isolated and structurally characterized according to methods well known to a person skilled in the art. Antibody A (HC SEQ ID NO 001, LC SEQ ID NO 002) is a high-affinity anti-IL-2 antibody and Antibody B (HC SEQ ID NO 003, LC SEQ ID NO 004) is a low-affinity anti-IL-2 antibody. Antibody C has no IL-2 affinity and has been included as a control. The genes encoding for VL-CL (light chain) and VH-CH1-CH2-CH3 (heavy chain) were cloned into the mammalian expression vector pcDNA3.4 into separate plasmids. The antibodies were produced using transient gene expression in Expi293 cells (Gibco, A14527) following standard protocols provided by the vendor. Plasmid DNA (HC/LC ratio 1:2 w/w) was transfected using ExpiFectamine^{™} 293 Reagent. The cells were maintained at 37°C, 8% CO2 in an orbital shaker (150rpm) for six days. The antibodies were purified to homogeneity from the supernatant by Protein A Chromatography (MabSelect^{™} SuRe^{™}, GE17-5438-01). Quality control of the proteins was performed by SDS-PAGE (NuPAGE^{™} 4-12% Bis-Tris Protein Gels , ThermoFisher) and analytic size exclusion chromatography (SEC) (GE lifesciences, Superdex 200 increase 10/300). Yields and purity are reported in Table 1. All antibodies were obtained as pure protein product, eluting as single peak in SEC-HPLC analysis.

Binding to IL-2 was first tested by ELISA using a serial dilution of antibodies on coated IL-2. Recombinant human IL-2 was coated on Maxisorp ELISA plates (Invitrogen, 44-2404-21) at 5µg/ml, 4°C overnight. After 2 hours blocking, antibodies A, B and C were incubated in a serial dilution starting from 10 µg/ml in assay buffer. The antibodies were detected with anti-human IgG-Peroxidase (Sigma, A0170). After addition and blockade of the chemiluminescent substrate, absorption was read (A₄₅₀-A₅₇₀ at plate reader, Spectramax iD3). EC₅₀ values were obtained by plotting Absorbance vs. log(Concentration) and sigmoidal 4-PL fitting using GraphPad Prism (Table 1). Only Antibody A displayed binding to IL-2 in ELISA and could be detected on the Fc-portion by an anti-human IgG antibody coupled to HRP. Binding affinities to recombinant human IL-2 (rhIL-2) of Antibodies A, B and C of the present invention were then measured using more sensitive biolayer interferometry (BLI) methodology. Antibodies were immobilized on amine reactive (2^{nd} Generation) sensors (ForteBio, 18-5092). Association (600s) and dissociation (900s) of a dilution series of recombinant IL-2 (Aero Biosystems, IL2-H4113) were measured on the antibody-coated biosensors on an Octet -System (Octet RED, ForteBio). K_{D} values were obtained by fitting the kinetics data with the ForteBio Data Analysis Software (8.2). This assay confirmed Antibody A binds rhlL-2 with high affinity. Binding of Antibody B could not be measured by ELISA, but a weak affinity constant could be determined by BLI. Antibody C did not show binding to rhlL-2 up to concentrations ≥1000 nM (Table 1).

**Table 1 Production yields from transient expression, purity and binding affinities to recombinant hIL-2 measured by ELISA or BLI.**

| **Antibodies** | **Yield (mg/ml)** | **Purity by SEC-HPLC (%)** | **EC₅₀ to IL-2 by ELISA (nM)** | **K_{D} to IL-2 by BLI (nM)** |
|---|---|---|---|---|
| A | 193.9 | 95.8% | 0.37 | 2.1 |
| B | 282.4 | 98.9% | - | 269.8 |
| C | 453.5 | 98.5% | - | - |

Fusion proteins comprising an IL-2 polypeptide, or an IL-2 mutein comprising amino acid substitutions with favorable pharmacokinetic properties (Table 2), have previously been demonstrated to provide IL-2 signaling when conjugating the cytokine to an antibody chain by C-terminal linkage to an antibody variable chain region (see for example, WO2018184964A1; Deak LC. et al. 2022, Nature 610:161; Gutbrodt KL. 2013 Sci. Trans. Med. 5:201; Gillies SD. (1992) PNAS 89(4):1428), or N-terminal linkage to an anti-IL-2 antibody (WO2017122130A1).

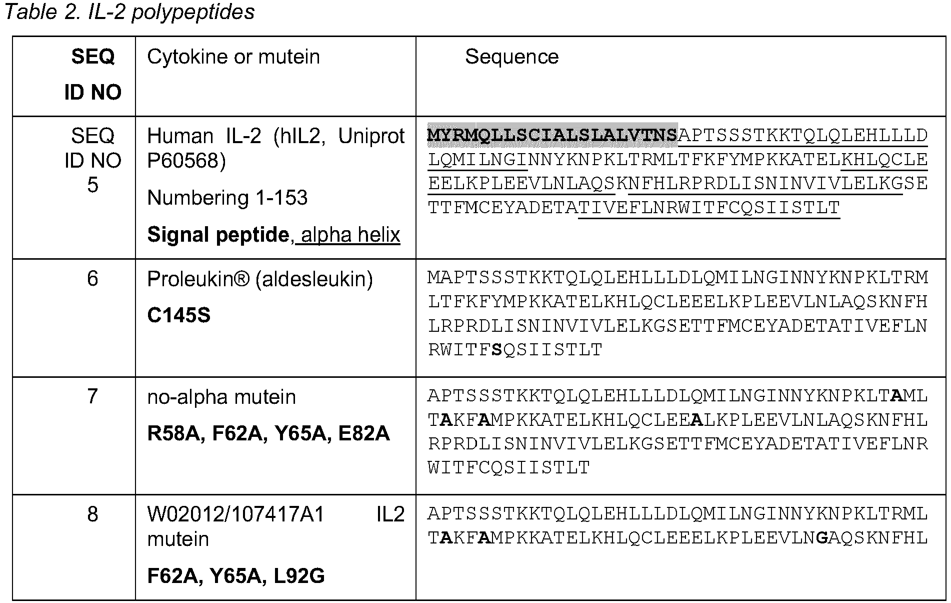

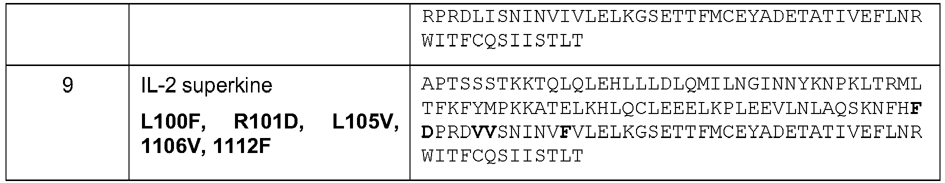

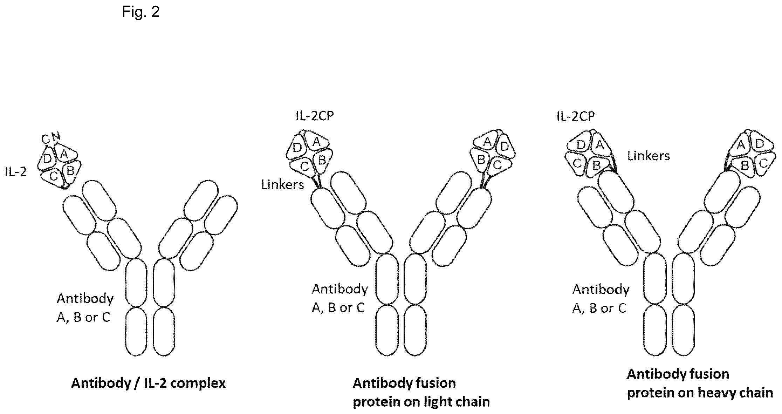

The inventor's assessed an alternative format, analyzing the structure of IL-2 in complex with the antibody NARA1 (RCSB Protein data bank 5LQB) from which humanized Antibody A and Antibody B were derived, to guide the embedding or fusion of IL-2 to preserve orientation of the cytokine relative to the antibody and similar receptor signaling qualities (Arena-Ramires et al. 2016 Sci. Trans. Med. 8:367). Based on the crystal structure of the NARA1/IL-2 complex, possible sites for embedding were identified by checking connecting strands between the different alpha helix domains (assigned here as A-D) and their proximity to CDR or framework regions on the VH or VL of the antibody. Various combinations of circularly permuted IL-2 (IL-2CP) were designed, to retain the cytokine's tertiary structure and orientation with respect to natural binding to the antibody, when fused to the indicated heavy chain or light chain variable regions (Table 3, Figure 2). Depending on the juncture site, the IL-2 needs to be permuted differently, in order to maintain the same orientation on the antibody. To create a circular permutation with the IL-2 helix domains CDAB format, the crystal structure suggested the region V89 to D104 (referring to the sequence of Proleukin) between the Band C helix could be opened to maintain key tertiary structures. To create a circular permutation with the IL-2 helix domains BCDA format, the crystal structure suggested the region G47 to E72 between the A and B helix could be opened. To create a circular permutation with the IL-2 helix domains DABC format, the crystal structure suggested the region G118 to I134 between the C and D helix could be targeted.

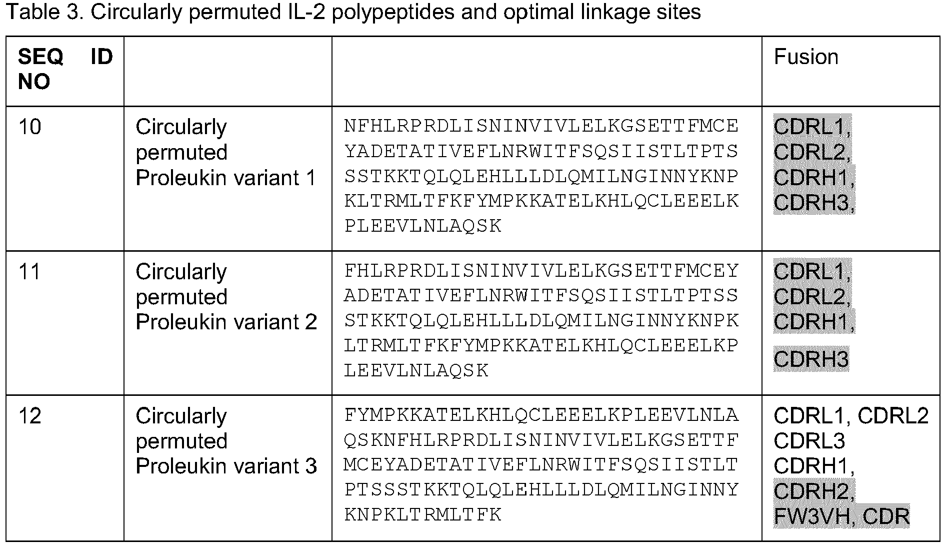

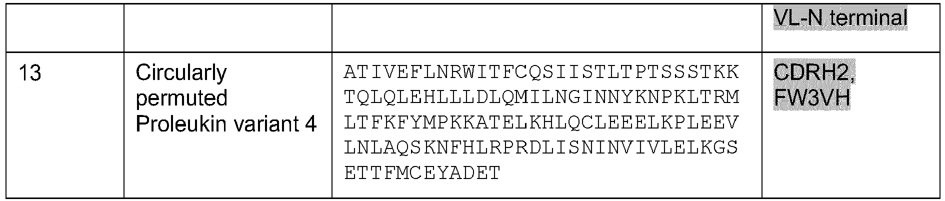

Representative IL-2 fusion proteins were then designed according to some embodiments of the present invention comprising Antibodies A, B or C joined to IL-2 either directly, or by means of linkers of various lengths as set out in Table 4.

The LCDR1 of the IL-2 Antibody A Kabat residues Y27d-D30 (or Y31-D34 structure numbering), and the region connecting IL-2 helix B and helix C between residue S95 and N97 were identified as promising regions for further engineering. The LCDR1 of each Antibody A was opened between Y27d and D30 numbered according to Kabat definition, to provide new C-terminal residue at LCDR1 Y27d, and a new N-terminal LCDR1 D30. LCDR1 residue Y27d was joined to the N-terminus of an IL-2CP opened between K96 and N97 (SEQ ID NO 10). The C-terminus of IL-2CP was joined to the LCDR1 residue 30 by the Kabat definition, such that residues Q28 and G29 of LCDR1 of the antibody were replaced by the circularized IL-2CP SEQ ID NO 10, joined directly, or by means of peptide linkers of various lengths. This embedding was repeated for equivalent amino acid residues with LCDR1 of the lower affinity IL-2 Antibody B, and Antibody C having no affinity for IL-2 (Table 4).

Further constructs were designed to validate an alternative IL-2CP insertion in the HDCR3, or HCDR2 region. VBE401 was developed using SEQ ID NO 011, an IL-2 opened between N97 and K96, inserting the cytokine (removing N97 and leaving F96 as new N-terminal end). SEQ ID NO 011 was inserted between residues E98 and G99 in the HCDR3 of Antibody A preceded by the linker GGG, and followed by the linker GGG. In addition, insertion of an IL-2CP into HDRC2 was tested. A circularly permuted IL-2 was created by opening the sequence between K63 and F64, and fusing the original N- and C-termini to provide SEQ ID NO 012. SEQ ID NO 012 was inserted between Antibody A HDCR2 residues G53 and S54, preceded by the linker GG, and followed by the linker GGG as described above (LIZ707).

For each structure (Table 4), the genes encoding VL-(IL-2)-CL, VL-CL, VH-CH1-CH2-CH3, VH-(IL-2)-CH1-CH2-CH3 were cloned into the mammalian expression vector pcDNA3.4 into separate plasmids. The antibody IL-2 fusion proteins were produced using transient gene expression in Expi293 cells as described for antibodies A, B and C above. The purity of all constructs was high (>95%), eluting as single peak in SEC-HPLC analysis (Table 5).

**Table 4. Antibody-IL-2 fusion proteins, with IL-2CP fusion site on the respective CDR of antibodies A, B or C. IL-2CP sequence is embedded within the variable domain of the heavy or light chain of the antibody as indicated. All heavy chains constant regions were SEQ ID NO 048, all light chain constant regions were SEQ ID NO 049.**

| Compound | IL-2 fusion site, Antibody clone | **IL2-CP (SEQ ID NO)** | Linker 1 (SEQ ID NO) | Linker 2 (SEQ ID NO) | VL (SEQ ID NO) | VH (SEQ ID NO) |
|---|---|---|---|---|---|---|
| EAD406 | CDR-L1, A | 010 | 0 AA | 1AA (014) | 025 | 043 |
| XFO227 | CDR-L1, A | 010 | 2 AA (015) | 3AA (016) | 026 | 043 |
| QTY065 | CDR-L1, A | 010 | 3 AA (016) | 4AA (017) | 027 | 043 |
| FJC828 | CDR-L1, A | 010 | 4 AA (017) | 5AA (018) | 028 | 043 |
| PGO345 | CDR-L1, A | 010 | 6 AA (019) | 7AA (020) | 029 | 043 |
| DRV470 | CDR-L1, A | 010 | 13AA (021) | 14AA (022) | 030 | 043 |
| XUB802 | CDR-L1, A | 010 | 19AA (023) | 20 AA (024) | 031 | 043 |
| EPK959 | CDR-L1, B | 010 | 0 AA | 1AA (014) | 032 | 044 |
| GYG794 | CDR-L1, B | 010 | 2AA (015) | 3 AA (016) | 033 | 044 |
| DRO069 | CDR-L1, B | 010 | 3AA (016) | 4 AA (017) | 034 | 044 |
| ECV200 | CDR-L1, B | 010 | 6 AA (019) | 7 AA (020) | 035 | 044 |
| BFC885 | CDR-L1, B | 010 | 13 AA (021) | 14AA (022) | 036 | 044 |
| LPT269 | CDR-L1, C | 010 | 0 AA | 1 AA (014) | 037 | 045 |
| DXM339 | CDR-L1, C | 010 | 2 AA (015) | 3 AA (016) | 038 | 045 |
| FUE433 | CDR-L1, C | 010 | 3 AA (016) | 4 AA (017) | 039 | 045 |
| LQM346 | CDR-L1, C | 010 | 6 AA (019) | 7 AA (020) | 040 | 045 |
| GLK754 | CDR-L1, C | 010 | 13AA (021) | 14AA (022) | 041 | 045 |
| VBE401 | CDR-H3, A | 011 | 3 AA (016) | 3 AA (016) | 042 | 046 |
| LIZ707 | CDR-H2, A | 012 | 2 AA (015) | 3 AA (016) | 042 | 047 |

### Immunoconjugate interactions with the CD132-CD122 heterodimeric receptor

The anti-IL-2 antibody clone 5344, binding on the CD122 binding site on IL-2, was used in order to determine correct folding of the IL-2 portion of the fusion proteins. The EC₅₀ values are reported in Table 6. The IL-2 moiety of all the constructs was successfully binding to the anti-IL-2 antibody (clone 5344) in ELISA and could be detected on the Fc-portion by an anti-human IgG antibody coupled to HRP (Table 5).

Binding to the CD122/CD132 complex (His-tagged, Acro Biosystem, Cat # ILG-H5283) of one representative anti-IL-2 antibody / IL-2 fusion (QTY065) was additionally assessed by SPR (BIAcore 3000, v4.1.2; GE Healthcare) and compared to Proleukin. CD122/CD132 was captured on an NTA sensor chip (GE Healthcare). Proleukin or QTY065 Fab were used as analytes. The calculated affinity constant for QTY065 Fab was 0.32 nM, comparable to the value of 0.42 nM obtained with Proleukin. The binding to the intermediate affinity IL-2R complex (CD122/CD132) remains unchanged in the IL-2CP antibody fusion proteins.

**Table 5. Purity of the final protein by SEC-HPLC and assessment of functionality by ELISA and CD25 binding by SPR.**

| Compound | **Purity SEC-HPLC (%)** | **EC₅₀ to 5344 ELISA (nM)** | **KD to CD25 SPR (nM)** |
|---|---|---|---|
| EAD406 | 97.61 | 0.11 | - |
| XFO227 | 97.12 | 0.14 | - |
| QTY065 | 99.10 | 0.14 | - |
| FJC828 | 97.77 | 0.28 | - |
| PGO345 | 95.75 | 0.16 | - |
| DRV470 | 96.85 | 0.10 | - |
| XUB802 | 94.58 | 0.15 | - |
| EPK959 | 99.20 | 0.15 | - |
| GYG794 | 97.80 | 0.20 | - |
| DRO069 | 97.52 | 0.11 | - |
| ECV200 | 96.80 | 0.13 | - |
| BFC885 | 97.74 | 0.25 | 54.1 |
| LPT269 | 97.47 | 0.24 | 0.12 |
| DXM339 | 97.93 | 0.28 | 0.18 |
| FUE433 | 98.61 | 0.54 | 0.16 |
| LQM346 | 99.09 | 0.28 | 0.15 |
| GLK754 | 98.61 | 0.28 | 0.20 |
| VBE401 | 92.55 | n.d. | - |
| LlZ707 | 91.55 | n.d. | - |

### Binding affinities to CD25 measured by surface plasmon resonance (SPR) analysis

To provide optimal biased signaling to CD8 T cells, CD25 binding of IL-2 fusion proteins is preferably minimal. In order to understand if the CD25 binding site on the antibody IL-2 fusion proteins was accessible, binding to CD25 was assessed by surface plasmon resonance (SPR) analysis (BIAcore 3000, GE Healthcare, 33-1140587-3682). His-tagged recombinant CD25 was captured via TrisNTA Biotin on SA chips, and kinetic titration of IL-2 fusion proteins of the present invention was performed reaching concentrations up to 500 nM. IL-2 fusion proteins to Antibody A did not display binding to CD25 up to concentrations ≤ 500 nM. Antibody A binds IL-2 with high affinity on the CD25 binding site, thereby blocking IL-2 binding to the receptor CD25. Antibody B binds IL-2 with low affinity and allows IL-2 binding to CD25 only when fused to IL-2 with long linkers (≥13 and 14 amino acids). Antibody C has no affinity for IL-2 and allows CD25 binding of its fused IL-2 with any linker length (Table 4 and 5).

### pSTAT5 in mouse splenocytes (EC50 of Tregs, CD8, NK)

STAT5 phosphorylation was analyzed in murine splenocytes as downstream signaling of the IL-2R activation. In order to assess the *in vitro* selectivity of the antibody-IL-2 fusion proteins, pSTAT5 was measured in different cell populations after stimulation with Proleukin, or with the compounds of the present invention. Freshly isolated murine splenocytes from C57BL/6 mice were incubated with a dilution series of Proleukin or IL-2-antibody fusion protein, starting from 100 nM. The cells were immediately fixed and stained with surface markers (i.e., CD25, CD3, NK1.1, CD4, CD8). After permeabilization (Perm III buffer, BD Biosciences), intracellular staining was performed (FoxP3, pSTAT5) before acquisition through Flow Cytometry. % of pSTAT5+ cells of CD8⁺ T cells, NK cells and CD4⁺CD25⁺FoxP3⁺ Tregs were plotted for each compound against molar concentration of IL-2 antibody fusion protein. EC50 values were calculated with GraphPad Prism (Table 6). Potency on Treg cells of IL-2 fusion proteins to Antibody A is markedly decreased compared to Proleukin, while the EC50 values on NK and CD8 T-cells are comparable. Fusion proteins to antibody B show reduced potency on Treg cells compared to Proleukin, although the biased effect gets reduced with increasing linker length (compound BFC885). IL-2 fused to Antibody C signals with high potency to Treg cells, comparable to Proleukin. Affinity of the antibody to the permuted IL-2 is required in order to efficiently provide steric hinderance and thereby exclude the CD25 from the signaling complex (Table 6).

**Table 6. EC50 of STAT5 activation of mouse splenocytes of IL-2-antibody fusion proteins on NK cells, CD8 cells, and Treg cells.**

| **Compound name** | **EC₅₀ NK cells (nM)** | **EC₅₀ CD8 cells (nM)** | **EC₅₀ Treg cells (nM)** |
|---|---|---|---|
| Proleukin | 1.4 | 4.1 | <0.016 |
| EAD406 | 5.2 | 15.7 | 22.0 |
| XFO227 | 2.7 | 8.0 | 10.6 |
| QTY065 | 1.2 | 4.6 | 5.91 |
| FJC828 | 1.9 | 5.8 | 8.4 |
| PGO345 | 2.3 | 6.6 | 7.6 |
| DRV470 | 2.4 | 7.9 | 10.3 |
| XUB802 | 5.8 | 17.1 | 12.1 |
| EPK959 | 1.9 | 16.0 | 16.0 |
| GYG794 | 1.5 | 18.6 | 4.8 |
| DRO069 | 0.9 | 6.8 | 6.6 |
| ECV200 | 0.9 | 6.9 | 3.7 |
| BFC885 | 2.0 | 26.4 | 0.9 |
| LPT269 | 1.7 | 32.0 | <0.016 |
| DXM339 | 1.3 | 18.6 | <0.016 |
| FUE433 | 1.2 | 15.7 | <0.016 |
| LQM346 | 1.8 | 25.4 | <0.016 |
| GLK754 | 2.5 | 70.5 | <0.016 |
| VBE401 | 34.3 | 32.2 | 2.6 |
| LIZ707 | 5.3 | 12.5 | 3.8 |

Applying the representative embedding procedures to IL-2-specific antibodies A or B resulted in fusion proteins with equally favorable IL-2 absence of CD25 binding (Table 5) and reduced potency in STAT5 phosphorylation on Treg cells compared to Proleukin (Table 6). This confirmed that by adjusting the orientation of the circularized IL-2 protein to conserve the natural binding orientation to an anti-IL-2 antibody to the cytokine, fusion to the heavy, or light chain variable region is feasible. A low affinity for IL-2 of antibody B (269 nM K_{D}) was sufficient to confer selective function of the resulting fusion protein. Peptide linkers of up to 20 amino acids in length retained desirable signaling qualities for the IL-2 fusion protein with Antibody A, though shorter peptides were preferred in combination with a lower affinity antibody. Fusion protein embedding IL-2CP in Antibody C did not exclude CD25 binding (even with 0-1 amino acid linkers), indicating that antibody affinity at least equivalent to Antibody B to IL-2 is required.

### Example 2: Generation of high affinity non-blocking anti-PD-1 antibodies

### Identification of anti-hPD-1 antibodies, non-competing with PD-1 agonists

Three anti-human PD-1 antibodies that were described as non-competing with PD-1 antagonists or non-PD-L1 blocking were identified (Table 7). These antibodies were tested for competition with commercial PD-1 antagonistic antibodies by flow cytometry using 20x molar excess of competitor. Raji PD-1 expressing cells (Invitrogen) were incubated 30 minutes at 4°C with serial dilutions of Pembrolizumab and Nivolumab, starting from 81 µg/ml (1:3 serial dilution). After washing the cells, antibodies in the first column of Table 8 labeled with biotin were added at a fixed concentration of 2 µg/ml. Bound antibodies were detected with Streptavidin-PE (Biolegend) and MFI levels of bound compound were compared to MFI in samples without competitor to determine the % signal inhibition. Background of samples incubated with Streptavidin-PE only was subtracted from all samples. Antibody XVT458 did not show any competition with any of the PD-1 antagonists tested. Antibody ZJN296 showed partial competition with Pembrolizumab, with a reduced mean fluorescence intensity (MFI) compared to sample with no competitor of 47.4%. Nonetheless, since the signal did not show a concentration dependent decrease, the reduced MFI in the sample with 20x competitor concentration may be unrelated to the presence of Pembrolizumab. Nivolumab did not significantly decrease the signal of antibody ZJN296. Full competition between antibody OVL714 was observed with both Pembrolizumab and Nivolumab, indicating a possible common epitope on the antigen.

**Table 7. Antibodies described as non-competing with PD-1 antagonists or PD-L1 and source. Percent inhibition of binding signal to cells expressing human PD-1 when pre-coated with 20x molar excess of the indicated commercial PD-1 antagonist antibodies.**

| Antibody | Species | Source | Pembrolizumab (% inhibition) | Nivolumab (% inhibition) |
|---|---|---|---|---|
| XVT458 | Humanized | (Clone NB01a) Fenwick C., J Exp Med, 2019 216(7):1525 | 5.6 | 0 |
| ZJN296 | Mouse | (Clone SJL-565-4) Adler AS., mAbs, 2017 | 47.4 | 12.9 |
| OVL714 | Mouse | (Clone SJL-566-3) Adler AS., mAbs, 2017 | 99.5 | 99.3 |

### Re-humanization and affinity maturation of antibody XVT458

The humanized VH sequence of XVT458, composed of shuffled frameworks from different families of germline genes, was re-humanized in order to obtain a VH with harmonic germline (GH), to provide z0-XVT458 (SEQ ID NOS 053, 054). Re-humanization of VH of XVT458 was performed by CDR grafting onto human IGHV-18*01 IGHJ6*01 framework, which showed highest homology to the framework regions of the parental construct. Mutations of residues of the vernier zone were inserted in order to observe impact on affinity to the hPD-1 antigen. Germline (GH wild type or with point mutations) and parental humanized (uVH) were combined with the humanized VL (uVL) in a full hIgG1 format (Table 8).

**Table 8. Derivatives of the anti PD-1 antibody XVT458 with newly humanized VH and binding kinetics measured by SPR.**

| Analyte | Ligand | VH | VL | kₐ (1/Ms) | k_{d} (1/s) | K_{D} (M) |
|---|---|---|---|---|---|---|
| hPD-1 Avi-Tag Biotin | XVT458 | uVH | uVL | 8.30E+04 | 1.33E-03 | 1.60E-08 |
| | z0-XVT458 | GH | uVL | 9.11E+04 | 1.47E-03 | 1.61E-08 |
| | z1-XVT458 | GH+V067A | uVL | 9.23E+04 | 1.46E-03 | 1.59E-08 |
| | z2-XVT458 | GH+T071V | uVL | 9.07E+04 | 1.27E-03 | 1.40E-08 |
| | z3-XVT458 | GH+T073K | uVL | 8.74E+04 | 1.42E-03 | 1.62E-08 |
| | z4-XVT458 | GH+V067A+T071V | uVL | 9.88E+04 | 1.31E-03 | 1.33E-08 |
| | z5-XVT458 | GH+V067A+T073K | uVL | 9.14E+04 | 1.45E-03 | 1.59E-08 |
| | z6-XVT458 | GH+T071V+T073K | uVL | 9.20E+04 | 1.33E-03 | 1.45E-08 |
| | z7-XVT458 | GH+V067A+T071V+T073K | uVL | 9.57E+04 | 1.36E-03 | 1.43E-08 |
| | z8-XVT458 | GH+V067A+T071V+T073K | mVL. | No or weak binding | | |
| cynoPD-1 His | XVT458 | uVH | uVL | 5.57E+04 | 3.73E-03 | 6.70E-08 |
| | z0-XVT458 | GH | uVL | 6.32E+04 | 3.58E-03 | 5.67E-08 |
| | z1-XVT458 | GH+V067A | uVL | 6.27E+04 | 3.59E-03 | 5.73E-08 |
| | z2-XVT458 | GH+T071V | uVL | 5.72E+04 | 3.10E-03 | 5.42E-08 |
| | z3-XVT458 | GH+T073K | uVL | 5.74E+04 | 3.31E-03 | 5.77E-08 |
| | z4-XVT458 | GH+VO67A+TO71V | uVL | 6.33E+04 | 3.23E-03 | 5.10E-08 |
| | z5-XVT458 | GH+V067A+T073K | uVL | 5.86E+04 | 3.38E-03 | 5.73E-08 |
| | z6-XVT458 | GH+T071V+T073K | uVL | 5.84E+04 | 3.14E-03 | 5.38E-08 |
| | z7-XVT458 | GH+V067A+T071V+T073K | uVL | 5.89E+04 | 3.21E-03 | 5.44E-08 |
| | z8-XVT458 | GH+V067A+T071V+T073K | mVL | No or weak binding | | |

### Determination of K_{D}:

The binding of each to PD-1 was assessed by SPR (Biacore 8K, GE Healthcare). Anti-human Fc IgG (Jackson) was immobilized on amine reactive CM5 chips (GE Healthcare) using 1xHBS-EP+ running buffer (GE Healthcare). The test antibodies were captured at flow rate 10 µl/min with 30 s contact time. Anti-PD-1 to human PD-1 (hPD-1) (AcroBiosystems, PD1-H82E4) and cynomolgus primate (cyno) PD-1 (cynoPD-1) (AcroBiosystems, PD1-C5223) were run as analytes, diluted in 1xHBS-EP+ running buffer (GE Healthcare) with 240 s association and 600 s dissociation at flow rate 30 µl/min. Regeneration was carried out with 10 mM Glycine-HCl, pH 1.5 at flow rate 10 µL/min. This protocol is to be used, unless stated otherwise, in determination of Kd whereever used to define the K_{D} of antibodies specified herein, with adaptions where necessary with regard to the nature of the interaction partners.

Binding of the antibody candidates on cell surface expressed antigen was confirmed by flow cytometry. CHO-S hPD-1 cells were incubated with a 5-fold serial dilution of the indicated humanized antibodies. Antibody binding was detected with goat anti-human IgG-PE. MFI were plotted over the antibody concentration to obtain EC50 values. Binding kinetics on SPR, and FACS EC50 binding values and maximal MFI values, indicated antibodies bound to hPD-1 expressing cells, except z8-ulgGKV326 (Tables 8 and 9).

**Table 9. Top MFI values and EC50 values of anti-PD-1 antibodies binding to hPD-1 expressed on CHO-S cells.**

| Ligand | Top MFI | FACS EC50 nM |
|---|---|---|
| XVT458 | 45100 | 8.60 |
| z0-XVT458 | 45185 | 5.74 |
| z1-XVT458 | 45235 | 5.85 |
| z2-XVT458 | 45528 | 4.15 |
| z3-XVT458 | 46192 | 5.47 |
| z4-XVT458 | 46726 | 4.54 |
| z5-XVT458 | 46173 | 4.51 |
| z6-XVT458 | 46791 | 3.93 |
| z7-XVT458 | 45991 | 3.88 |
| z8-XVT458 | 0 | NA |
| IgG1 isotype control | 0 | NA |

The humanized antibody z2-XVT458 (SEQ ID NOS 055, 056) was affinity matured by inserting random mutations in the CDRs (parsimonious mutagenesis method). Every position in the antibody VH and VL-CDRs was mutated by PCR using a mutagenic primer containing a degenerate codon NNS at a specific CDR position, introducing a combination of all 20 amino acids. The library of single mutants was screened by scFv capture ELISA using a redundancy factor of 4 (on 96-well plate er position). Clones displaying ≥ 2X ELISA signal compared to the wild-type scFv were sequenced, and clones with unique sequences were re-grown, re-screened by capture ELISA and ranked by dose-dependent ELISA on human and cyno antigens, and by FACS using human antigen expressing cells. Affinity improving mutations were used to design a combinatorial library using the Kunkel method. The library was screened by scFv capture ELISA using the redundancy factor of 4 (4x number of combinations). Clones displaying ≥ 2X ELISA signal compared to the wild-type scFv were sequenced, and clones with unique sequences were re-grown, re-screened by capture ELISA and ranked by dose-dependent ELISA on human and cyno antigens, and by FACS using human antigen expressing cells. Primary screening by saturated mutagenesis of antibody CDRs identified 25 mutants at 10 CDR amino acid positions that showed capture ELISA signal at least 2-fold higher than wild-type on hPD-1. 9 mutants showed improved binding to both recombinant hPD-1 and cynoPD-1, and hPD-1 on CHO-S cells. A combinatorial library was generated with these 9 mutants yielding 16 combinatorial variants with improved affinity to recombinant and cell-expressed antigen. From the base sequence of z2-XVT458 (variable domains SEQ ID NOs 055, 056), 6 subclones, z2-XVT458 m1-m6 (SEQ ID NOS 057 to 068), were selected for IgG conversion. In addition to the CDR mutations, the sequences of z2-XVT458-m1, z2-XVT458-m3 and z2-XVT458-m6 contain a G57D mutation just after the CDRL2, which may contribute to the increased affinity of these clones. The selected clones in IgG format showed a significant affinity improvement to recombinant hPD-1, cynoPD-1 and hPD-1 on cells. Compared to parental z2-XVT458, these clones (z2-XVT458m1-m6) also displayed 10-84 folds improvement in k_{off} rate as measured by SPR on both hPD-1 and cynoPD-1 (Table 10; corresponding Seq ID NOs 057-068). For SPR the same method as above was used.

**Table 10. Parental XVT458, z2-XVT458 and subclone affinity matured mutants in IgG format. Binding affinities measured by flow cytometry, ELISA and SPR.**

| **Abs** | **ELISA Binding** | | | |
|---|---|---|---|---|
| | **hPD-1** | | **cynoPD-1** | |
| | **EC50 (nM)** | **Max OD** | **EC50 nM(nM)** | **Max OD** |
| **XVT458** | 1.04 | 2.85 | 0.5 | 3.21 |
| **XVT458-z2** | 0.71 | 2.91 | 0.4 | 3.32 |
| **XVT458-z2-m1** | 0.083 | 3.39 | 0.087 | 3.58 |
| **XVT458-z2-m3** | 0.089 | 3.37 | 0.094 | 3.54 |
| **XVT458-z2-m5** | 0.051 | 3.42 | 0.056 | 3.58 |
| **XVT458-z2-m6** | 0.064 | 3.49 | 0.071 | 3.69 |
| **XVT458-z2-m4** | 0.079 | 3.3 | 0.098 | 3.54 |
| **XVT458-z2-m2** | 0.076 | 3.34 | 0.086 | 3.56 |
| **NC.hIgG1** | >50 | 0.05 | >50 | 0.07 |

| **Abs** | **FACS Binding** | | | |
|---|---|---|---|---|
| | **CHO-S hPD-1** | | **CHO-S Cyno-PD-1** | |
| | **EC₅₀ (nM)** | **Max MFI** | **EC₅₀ (nM)** | **Max MFI** |
| **XVT458** | 6.73 | 39900 | NA | 52 |
| **XVT458-z2** | 5.86 | 40700 | NA | 109 |
| **XVT458-z2-m1** | 1.5 | 41000 | NA | 70 |
| **XVT458-z2-m3** | 1.34 | 41100 | NA | 124 |
| **XVT458-z2-m5** | 0.84 | 42400 | NA | 335 |
| **XVT458-z2-m6** | 1.56 | 39500 | NA | 59 |
| **XVT458-z2-m4** | 0.96 | 43400 | NA | 258 |
| **XVT458-z2-m2** | 1.15 | 42800 | NA | 367 |
| **NC.hIgG1** | NA | 184 | NA | 206 |
| | | | | |

| **Abs** | | | **SPR** | |
|---|---|---|---|---|
| | **hPD-1** | | | |
| | **ka (1/Ms)** | **kd (1/s)** | **K_{D} (M)** | **ka (1/Ms)** |
| **XVT458** | 9.48E+04 | 1.22E-03 | 1.28E-08 | 6.90E+04 |
| **XVT458-z2** | 9.92E+04 | 1.24E-03 | 1.25E-08 | 7.30E+04 |
| **XVT458-z2-m1** | 2.22E+05 | 3.28E-05 | 1.48E-10 | 1.85E+05 |
| **XVT458-z2-m3** | 1.58E+05 | 6.57E-05 | 4.17E-10 | 1.38E+05 |
| **XVT458-z2-m5** | 1.24E+05 | 9.77E-05 | 7.87E-10 | 1.11E+05 |
| **XVT458-z2-m6** | 2.31E+05 | 1.01E-04 | 4.39E-10 | 2.13E+05 |
| **XVT458-z2-m4** | 1.42E+05 | 1.73E-04 | 1.22E-09 | 1.34E+05 |
| **XVT458-z2-m2** | 1.53E+05 | 1.90E-04 | 1.24E-09 | 1.27E+05 |
| **NC.hIgG1** | / | / | / | / |

### Humanization of antibody ZJN296

ZJN296 was humanized by CDR grafting using germlines with highest identity with the murine framework (IGKV1-33*01 IGKJ2 and IGHV1-18*01 IGHJ6*01). To increase the likelihood of retaining the binding affinity to the target antigen, 8 additional humanized VL-genes and 2 humanized VH-genes were designed by mutating human amino acids back to mouse. Such mutations may preserve the original conformation of VH and VL CDR loop and antigen binding if this loop makes contact with the antigen (Table 11).

**Table 11. Antibody variants of humanized ZJN296, with mutations inserted in humanized ZJN296-0 VH (GH, SEQ ID NO 069) and VL (SEQ ID NO 070).**

| **ID** | **VH** | **VL** | **ID** | **VH** | **VL** |
|---|---|---|---|---|---|
| ZJN296 | mVH | mVK | ZJN296-9 | GH+V002A | GL+S060D |
| ZJN296-0 | GH | GL | ZJN296-10 | GH+T071V | GL+S060D |
| ZJN296-1 | GH+V002A | GL | ZJN296-11 | GH+T073R | GL+S060D |
| ZJN296-2 | GH+T071V | GL | ZJN296-12 | GH+V002A+T071V | GL+S060D |
| ZJN296-3 | GH+T073R | GL | ZJN296-13 | GH+V002A+T073R | GL+S060D |
| ZJN296-4 | GH+V002A +T071V | GL | ZJN296-14 | GH+T071V+T073R | GL+S060D |
| ZJN296-5 | GH+V002A +T073R | GL | ZJN296-15 | GH+V002A+T071V+ T073R | GL+S060D |
| ZJN296-6 | GH+T071V+ T073R | GL | ZJN296-16 | GH+V002A+V067A+ T071V+T073R | GL+S060D |
| ZJN296-7 | GH+V002A +T071V+T0 73R | GL | ZJN296-17 | mVH | GL+S060D |
| ZJN296-8 | GH | GL+S06 0D | ZJN296-18 | GH+V002A+V067A+ T071V+T073R | mVL |

Recombinant humanized antibody variants of ZJN296 were expressed as human IgG1 in HEK293 cells. For SPR analysis (Biacore 8K, GE Healthcare) anti-human Fc IgG (Bethyl, A80-304P) was immobilized on CM5 chips (Cytiva) using 1xHBS-EP+ running buffer. Humanized anti-PD-1 antibodies were captured on the chip. hPD-1 and cynoPD-1 were run as analytes 1xHBS-EP+ running buffer at a flow rate of 30 µL/min for an association phase of 180 s, followed by 400 s dissociation. 10 mM glycine (pH 1.5) as regeneration buffer was injected to flow cells following every dissociation phase.

Based on SPR binding kinetics on human and cyno antigen and binding data to hPD-1 expressed on cells tested with supernatants, 5 best candidates were expressed recombinantly and a full SPR kinetics was performed (Table 12). ZJN296-0 and ZJN296-6 were selected as candidates since ZJN296-0 has minimum back mutations and comparable binding potency compared with the chimeric antibody, and ZJN296-6 has the best binding characteristics.

**Table 12. SPR kinetics results of humanized purified ZJN296 derived antibodies**

| **Sample Name** | **Bindi ing to Human PD1** | | | **Bind ing to Cyno P D1** | | |
|---|---|---|---|---|---|---|
| | **kₐ (1/Ms)** | **k_{d} (1/s)** | **k_{D} (M)** | **kₐ (1/Ms)** | **k_{d} (1/s)** | **k_{D} (M)** |
| ZJN296 | 7.71E+04 | 7.37E-04 | 9.56E-09 | 8.93E+04 | 1.15E-02 | 1.28E-07 |
| ZJN296-0 | 5.69E+04 | 4.95E-04 | 8.70E-09 | 6.15E+04 | 6.41E-03 | 1.04E-07 |
| ZJN296-2 | 5.65E+04 | 3.24E-04 | 5.72E-09 | 5.96E+04 | 4.33E-03 | 7.27E-08 |
| ZJN296-3 | 5.61E+04 | 3.69E-04 | 6.57E-09 | 4.76E+04 | 5.11E-03 | 1.07E-07 |
| ZJN296-6 | 6.26E+04 | 2.01E-04 | 3.22E-09 | 6.36E+04 | 2.79E-03 | 4.39E-08 |

### Non-blocking antibody hybridoma screening

High affinity binding antibodies specific for human PD-1 (hPD-1), cross-reactive with cynomolgus monkey PD-1 (cynoPD-1), but not blocking PD-L1 or PD-1 antagonist antibodies were generated by immunization of humanized mice (AlivaMab^{®} mice) and hybridoma technology. Two separate immunization campaigns (with 10 Kappa-Lambda mice in first campaign, 4 Kappa and 4 Lambda in second campaign) were performed by immunization with a combination of h/cyPD-1. Primary functional screening was performed using hPD-1 and cyPD-1 transfected HEK293 cells. Binding was assessed by flow cytometry. 31 plates of 384 wells were screened in the first campaign, and 15 plates of 384 wells were screened in the second campaign. In the second screen selected clones were initially tested from supernatants for competition with Nivolumab and Pembrolizumab, testing binding on antigen expressing cells by flow cytometry in presence of the competitor antibodies and detecting with an anti-mouse IgG antibody. Non-competitors were determined as MFI(+ competitor)/MFI(no competitor) > 0.6. Only the non-competing clones, that showed high affinity binding on BLI (KD < 1 nM) were expanded and purified. Out of all the screened clones, 7 clones fitting the desired binding profile were identified (Table 13, SEQ ID NOs 071 to 084 VH and VL of antibodies that were used with CH_{1,2,3} and corresponding CL(kappa) or CL(lambda)). Table 13 summarizes the binding properties to human and cyno PD-1. EC50 binding to HEK293 cells transfected with hPD-1 and cyPD-1 were measured by flow cytometry. Binding kinetics to the recombinant antigen was measured by BLI. Association (220s) and dissociation (480s) of a dilution series of recombinant hPD-1 and cynoPD-1 (100, 25, 6.25 nM, AcroBio) were measured on the antibody-coated biosensors (anti-human-IgG CH1 Biosensor, ForteBio) Octet-System (Octet RED, ForteBio). K_{D} values were obtained by fitting the kinetics data with the ForteBio Data Analysis Software (8.2). PBS was used as assay buffer (PBS 10mM Phosphate, 150mM NaCl - pH 7.4). For dissociation the biosensors were dipped into kinetic assay buffer (PBS 10mM Phosphate, 150mM NaCl - 0.1% BSA - 0.02% Tween - pH 7.4). Sequencing of the final clones revealed that clones 21A08, 22F13 and 25I20 used the same HC/LC V-regions and have the same CDR3s. Clones 20H02, 39F23, 40B20 and 56H02 showed multiple development liabilities in the CDR regions. Clone 21A08 was selected as the best clone for its affinity and purity from SEC-HPLC compared to the other sibling clones.

**Table 13. FACS EC50 binding to hPD-1 or cynoPD-1 expressing HEK293 cells, SPR binding kinetics on hPD-1 and cynoPD-1.**

| | **FACS EC50 (M)** | | **PD1-HIS** | | | **cynoPD-1-HIS** | | |
|---|---|---|---|---|---|---|---|---|
| **clone** | **293:huPD1** | **293:cyPD1** | **KD (M)** | **kdis(1/s)** | **kon(1/Ms)** | **KD (M)** | **kdis(1/s)** | **kon(1/Ms)** |
| **21A08** | 2.43E-10 | 1.95E-10 | 9.27E-12 | 6.39E-07 | 6.90E+04 | 1.24E-09 | 8.25E-05 | 6.67E+04 |
| **22F13** | 2.00E-10 | 1.07E-10 | 1.41E-09 | 1.32E-04 | 9.40E+04 | 8.45E-09 | 8.38E-04 | 9.92E+04 |
| **25I20** | 1.79E-10 | 3.10E-10 | 1.15E-09 | 1.01E-04 | 8.75E+04 | 8.38E-09 | 7.83E-04 | 9.34E+04 |
| **20H02** | 5.03E-10 | 2.31E-10 | 2.43E-09 | 2.21E-04 | 9.09E+04 | 6.39E-09 | 4.63E-04 | 7.25E+04 |
| **39F23** | 1.89E-11 | 2.89E-11 | 1.03E-09 | 2.53E-04 | 2.44E+05 | 1.30E-09 | 2.94E-04 | 2.27E+05 |
| **40B20** | 1.62E-10 | 9.58E-11 | 9.61E-09 | 7.73E-04 | 8.05E+04 | 7.51E-09 | 5.05E-04 | 6.73E+04 |
| **56H02** | 9.21E-11 | 8.81E-11 | 2.28E-10 | 3.79E-05 | 1.66E+05 | 2.62E-10 | 3.67E-05 | 1.40E+05 |

In order to remove an N-glycosylation site present in the CDR-L1, mutations were inserted in the CDR-L1 of 21A08. The first asparagine (Asp, N) in CDR-L1, was replaced by a serine (Ser, S), Glutamine (Gln, Q), or Alanine (Ala, A). Binding of the 21A08 clone variants (21A08S, 21A08Q and 21A08A) was tested by BLI and compared to the wt clone using supernatants of transiently transfected HEK293 cells, and a positive control (Pembrolizumab).

**Table 14. Antibody clone 21A08 and deglycosylated variants with binding affinity (KD) and dissociation constant (Koff).**

| Clone name | Mutations LCDR1 | KD huPD-1 (M) | Koff 1/s |
|---|---|---|---|
| 21A08 | - | 2.17 E-09 | 1.40 E-04 |
| 21A08S | N>S | 2.47 E-09 | 1.84 E-04 |
| 21A08Q | N>Q | 3.39 E-09 | 2.67 E-04 |
| 21A08A | N>A | 1.81 E-09 | 1.81 E-04 |
| Pembrolizumab | | 6.29 E-09 | 1.61 E-03 |

In addition, a deamidation site present in the CDR-L1 was removed by inserting the mutations listed in Table 15. Binding kinetics of 21A08A and the deamidation site removal mutants were measured by SPR. For SPR analysis (Biacore 8K, GE Healthcare) anti-human Fc IgG (Bethyl, A80-304P) was immobilized on CM5 chips (Cytiva) and the test anti-PD-1 antibodies were captured using 1xHBS-EP+ running buffer at flow rate 10 µL/min. hPD-1 and cynoPD-1 (His -tagged) were run as analytes diluted in 1xHBS-EP+ running buffer at a flow rate of 30 µL/min for an association phase of 250 s, followed by 3600 s dissociation. 10 mM glycine (pH 1.5) as regeneration buffer was injected to flow cells following every dissociation phase. All clones lacking the N-glycosylation site retained binding to PD-1 (Table 14). None of the mutations in the CDR-L1 compromised the binding kinetics to hPD-1 or cynoPD-1 significantly.

**Table 15. Antibody clone 21A08A and variants with deamidation site removed, binding affinity and dissociation constant**

| Clone name | Mutation LCDR1 | ka hPD-1 (1/Ms) | kd hPD-1 (1/s) | KD hPD-1 (M) | ka cPD-1 (1/Ms) | kd cPD-1 (1/s) | KD cPD-1 (M) |
|---|---|---|---|---|---|---|---|
| 21A08A | - | 6.3E+04 | <1.0E-05 | <1.6E-10 | 5.6E+04 | 9.6E-05 | 1.7 E-09 |
| 21A08Ap1 | NS>QS | 5.7E+04 | 2.9E-05 | 5.0 E-10 | 5.2E+04 | 1.6E-04 | 3.0 E-09 |
| 21A08Ap2 | NS>SS | 6.4E+04 | 4.2E-05 | 6.6 E-10 | 5.9E+04 | 1.9E-04 | 3.2 E-09 |
| 21A08Ap3 | NS>NA | 6.0E+04 | 3.5E-05 | 5.8 E-10 | 5.5E+04 | 2.5E-04 | 4.67E-09 |

### Example 3: binding profile of non-blocking anti-PD-1 antibodies

Assessment of the binding profile of non-blocking anti-PD-1 antibodies was then made using a human plasma membrane protein cell array. The Retrogenix Cell Microarray Technology platform screened the anti-PD-1 antibody candidates for cross-reactivity binding with non-target proteins. The test antibodies were each screened for binding against human HEK293 cells, individually expressing 6018 full-length human plasma membrane proteins, secreted and cell surface-tethered human secreted proteins plus a further 397 human heterodimers. In a pre-screen 2 µg/mL of each test antibody or PBS only was added to slides of fixed un-transfected HEK293 cells. Binding to un-transfected cells was assessed using an AlexaFluor647 labelled anti-human IgG Fc detection antibody (AF647 anti-hlgG Fc), followed by fluorescence imaging. In the pre-screen with antibody XVT458-z2-m5 a high background was detected. A second pre-screen was repeated with 0.5mg/ml, which resulted in background reduction. For library screening 6018 expression vectors, encoding both ZsGreen1 and a full-length human plasma membrane protein or a cell surface-tethered human secreted protein, were individually arrayed in duplicate across 17 microarray slides ('slide-sets'). In addition, vectors encoding a further 397 human heterodimers were co-arrayed across a further microarray slide. Human HEK293 cells were used for reverse transfection/expression. Test antibody XVT458-z2-m5 was added to each of the 18 slide-sets after cell fixation giving a final concentration of 0.5 µg/mL, the remaining 9 test antibodies were added at final concentration of 2 µg/mL. Detection of binding was performed by using the same fluorescent secondary antibody as used in the pre-screen (AF647 anti-hlgG Fc). Fluorescent images were analyzed and quantitated (for transfection) using ImageQuant software (GE healthcare, Version 8.2). A protein 'hit' is defined as a duplicate spot showing a raised signal compared to background levels. This is achieved by visual inspection using the images gridded on the ImageQuant software. Hits were classified as 'strong, medium, weak or very weak', depending on the intensity of the duplicate spots. The screen revealed 29 library hits. After removing 12 interactions which were observed with a test antibody and the control treatment and therefore designated as non-specific, a further 7 low confidence interactions, 10 specific interactions for the test antibodies were identified (Table 16). All test antibodies showed a single specific interaction with PD-1 (PDCD1). Test antibodies ZJN296-0, ZJN296-6, XVT458-z2-m1, XVT458-z2-m2, XVT458-z2-m4, XVT458-z2-m5 all showed medium to weak interactions with other target proteins. Clones 21A08Ap1 (SEQ ID NO VH 085, VL 086), 21A08Ap2 (SEQ ID NO VH 085, VL 087), XVT458-z2-m3 (SEQ ID NO 061, 062), and XVT458-z2-m6 (SEQ ID NO 067, 068) showed single specific interaction to the target of interest.

**Table 16. Hits from the Retrogenix cell microarray screen. Protein types are Plasma membrane (PM), Secreted (S), Tethered Secreted (TS), Heterodimer (HD) or Evidence of plasma membrane (M). Hits no., above very weak intensity.**

| **Sample Id and dose** | Hits | Hit no. | 1 | 2 | 3 | 4 | 5 |
|---|---|---|---|---|---|---|---|
| | | Gene ID | PDCD1 | RTN4RL2 | PROM2 | APP | SV2A |
| | | Protein Type | PM | PM | PM | PM | PM |
| | | Ref seq isoform (for | single form | Isoform 1 | Isoform 1 | Isoform APP751 | Isoform 1 |
| 21A08Ap1 (2 µg/mL) | 1 | | strong | | | | |
| 21A08Ap2 (2 µg/mL) | 1 | | strong | | | | |
| ZJN296-0 (2 µg/mL) | 2 | | strong | medium | | | |
| ZJN296-6 (2 µg/mL) | 2 | | strong | medium | | | |
| XVT458-z2-m1 (2 µg/mL) | 2 | | strong | | medium | | |
| XVT458-z2-m2 (2 µg/mL) | 6 | | strong | | medium | weak | weak |
| XVT458-z2-m3 (2 µg/mL) | 1 | | strong | | | | |
| XVT458-z2-m4 (2 µg/mL) | 4 | | strong | | | | |
| XVT458-z2-m5 (0.5 µg/mL) | 2 | | strong | | | | |
| XVT458-z2-m6 (2 µg/mL) | 1 | | strong | | | | |
| PBS (secondary only)* | | | | | | | |

| **Sample Id and dose** | Hits | Hit no. | 6 | 7 | 8 | 9 | 10 |
|---|---|---|---|---|---|---|---|
| | | Gene ID | DCC | RARRES2 | IGSFl | CD248 | NLGN1 |
| | | Protein Type | PM | TS | PM | PM | PM |
| | | Ref seq isoform (for | single form | single form | Isoform 1 | Isoform 1 | Isoform 2 |
| 21A08Ap1 (2 µg/mL) | 1 | | | | | | |
| 21A08Ap2 (2 µg/mL) | 1 | | | | | | |
| ZJN296-0 (2 µg/mL) | 2 | | | | | | |
| ZJN296-6 (2 µg/mL) | 2 | | | | | | |
| XVT458-z2-m1 (2 µg/mL) | 2 | | | | | | |
| XVT458-z2-m2 (2 µg/mL) | 6 | | weak | weak | | | v.weak |
| XVT458-z2-m3 (2 µg/mL) | 1 | | | | | | |
| XVT458-z2-m4 (2 µg/mL) | 4 | | | | weak | weak | weak |
| XVT458-z2-m5 (0.5 µg/mL) | 2 | | | weak | | | v.weak |
| XVT458-z2-m6 (2 µg/mL) | 1 | | | | | | |
| PBS (secondary only)* | | | | | | | |

### Example 4: Non-competitive binding of anti-PD-1 antibodies with PD-L 1 antagonists and PD-L1

### Competition on hPD-1 expressing cells

Antibodies 21A08Ap1 and 21A08Ap2 were tested for competition with commercial PD-1 antagonistic antibodies by flow cytometry. Jurkat PD-1 expressing cells were incubated 30 minutes at 4°C with a serial dilution of Pembrolizumab and Nivolumab, starting from 10 µM (1:3 serial dilution). Without washing the cells antibodies 21A08Ap1 and 21A08Ap2 labeled with biotin were added at a fixed concentration of 100 nM. Bound antibodies were detected with Streptavidin-PE (Biolegend) and MFI levels of bound compound were compared to MFI in samples without competitor to determine the % signal inhibition. Background of samples incubated with Streptavidin-PE only was subtracted from all samples. Inhibition was minimal, under 20%, showing neither Pembrolizumab nor Nivolumab significantly decreased the signal of antibodies 21A08Ap1 and 21A08Ap2 (Table 17).

**Table 17. Competition on hPD-1 expressed on cells with commercial PD-1 antagonists, Pembrolizumab and Nivolumab. Flow cytometry % signal inhibition of the tested antibody using 100x molar excess of competitor.**

| Antibody name | Signal inhibition in % with 100x molar excess | |
|---|---|---|
| | Pembrolizumab | Nivolumab |
| 21A08Ap1 | 11.1 | 7.3 |
| 21A08Ap2 | 4.4 | 0 |

### Simultaneous binding of hPD-1 to 21A08Ap1 and PD-L1

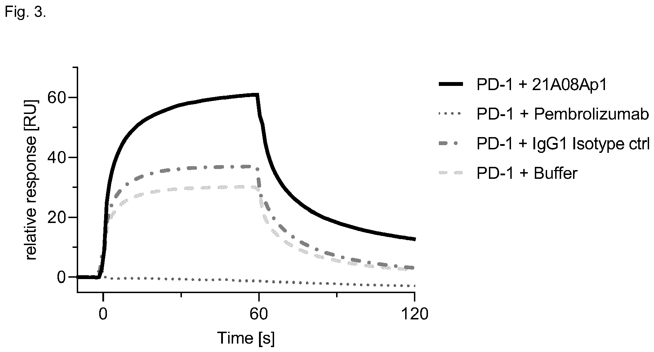

Simultaneous binding of PD-1 (Fc-Tag) to anti-PD-1 antibodies and PD-L1 (His-Tagged, Acrobiosystems, #H52H3) was assessed by SPR analysis using the Biacore T200 (Cytiva, #28975001). PD-L1 was captured on an anti-His-CM5 chip (Cytiva, chip: #29104988, His Capture Kit: #28995056). 1 µM of PD-1 was premixed with 5 µM of antibody (Pembrolizumab, IgG1 isotype control antibody, or 21A08p1) or running buffer (Xantec, HBSTE: B HBSTE10) for at least 30 min before binding analysis (association and dissociation 60s each). Data was analyzed using the Biacore Insight Evaluation Software (Cytiva, V4.0.8 # 29310606). Double reference subtraction was performed (surface without ligand and running buffer injection). Figure 3 reports the Biacore sensograms. No binding (as change in RU) was observed for PD-1 pre-mixed with Pembrolizumab, confirming that Pembrolizumab blocks the PD-L1 binding site of PD-1. By contrast, the complex of PD-1 with the antibody 21A08Ap1 was still able to bind to PD-L1, giving a signal of approximately 60 RU. The controls of PD-1 alone and PD-1+IgG1 isotype control showed an association signal of approximately 30 RU.

### Example 5: Rational design of non-blocking PD-1 targeted IL-2 fusion proteins

Proof-of-concept compounds were designed and characterized to determine the most favorable format for adding a non-blocking PD-1 targeting moiety to a CD122/CD132 dimeric receptor biased IL-2-anti-IL-2 antibody fusion moiety. The anti-PD-1 antibodies XVT458 or ZJN296 (Table 7) were combined with Antibody A - IL-2 fusion protein (QTY065, SEQ ID NO 051, 052) to generate bispecific compounds delivering specifically CD122-CD132 biased IL-2 to PD-1 expressing cells.

Five formats were designed different in size and valency to the PD-1 antigen (Fig. 4, Table 18). The double-scFv fusion comprises the anti-PD-1 antibody XVT458 in scFv format, i.e. the VH and VL domains fused by a 15 amino acid Glycine(G)-Serine(S) linker, fused to the QTY065 IL-2-antibody fusion scFv by a G4S linker (Fig. 4A). The Fab-double scFv consists of the QTY065 antibody-IL-2 fusion as Fab fragment (IL-2-VL-CL and VH-CH1) fused at each C-terminus to two anti-PD-1 scFv fragments of the XVT458 antibody (by their N-terminus VH-(G₄S)₃-VL, Fig. 4B). The IgG CrossMab format is a heterotetrameric human IgG1, with HC1 and LC1 from the anti-PD-1 antibodies XVT458 or ZJN296 and HC2 and LC2 from antibody-IL-2 fusion QTY065 (Fig. 4C). Knob into hole mutations (Y407T on HC1 and T366Y on HC2) were used to ensure correct heavy chain pairing. To enhance correct light chain pairing the CL and CH1 domains of HC1 and LC1 were swapped (WO2009080253A1). For Fc silencing of the IgG1 Fc region the mutations L234A, L235A, P329G (WO2012130831A1) were inserted on both HC1 and HC2. The IgG-scFv fusion proteins include the antibody XVT458 as full human IgG1 (consisting of HC1, identical LCs, HC2) having the HC2 fused on either its N-terminus (Fig. 4E) or C-terminus (Fig. 4D) to the IL-2-antibody fusion QTY065 as scFv (i.e., IL-2-VL_{AntibodyA}-(G₄S)₃ - VH_{AntibodyA}-(G₄S)₂ - HC2_{XVT458} or HC2_{XVT458}- (G₄S)_{2/4}-IL-2-VL_{AntibodyA}-(G₄S)₃ - VH_{AntibodyA}). Pairing to HC1 is enhanced by the knob into hole mutations (same as CrossMab) and Fc silencing by L234A, L235A, P329G mutations. The bispecific antibodies were produced in Expi297 cells as described for IL-2 fusion proteins described above.

The functionality of the bispecific antibody was assessed with a sandwich ELISA that relies on its binding to the target antigen (coated hPD-1) and the integrity of the fused IL-2 via a secondary antibody (anti-IL-2 clone 5344). 60 nM of hPD-1 (ECD-His, produced in house) was coated on a Maxisorp plate (Nunc) overnight at 4°C and blocked with 5% BSA in PBS. Bispecific antibodies were added in a serial dilution in assay buffer and detected with biotinylated-5344 and Streptavidin HRP (BD Pharmingen, 554066). Absorbance signal after adding TMB was read with a plate reader (Spectramax ID3) at 450 nm. EC50 values were determined by blotting absorbance against concentration (Graphpad Prism, sigmoidal curve fit, 4 PL, X-axis is log). All compounds showed binding on both bispecific moieties; formats with bivalent binding to hPD-1 showed lower EC50 values compared to the monovalent formats (Table 18). Binding to hPD-1 expressed on the surface of Jurkat-PD-1 cells was confirmed by flow cytometry (Table 18). All formats retained binding to hPD-1. As expected, compounds bearing two PD-1-binding domains showed increased binding to hPD-1 due to avidity effects.

**Table 18. Anti-PD-1, anti-hIL-2-IL-2 bispecific antibodies in various formats, with valency, molecular weight (Mw), EC50 to hPD-1 measured by ELISA and binding to hPD-1 expressed on Jurkat-hPD-1 cells measured by flow cytometry (MFI fold over background).**

| Compound ID | Anti PD-1 antibody | Format | Binding arms to hPD1 | Anti-hlL-2/ IL-2 arms | Mw (kDa) | EC50 (nM) | hPD-1 binding (fold) |
|---|---|---|---|---|---|---|---|
| HRL470 | XVT458 | Double scFv fusion | 1 | 1 | 67.7 | 1.24 | 41.9 |
| VNP090 | XVT458 | Fab- double scFv | 2 | 1 | 116 | 0.11 | 19.9 |
| FQQ111 | XVT458 | IgG format with CrossMab^{CH1-CL} | 1 | 1 | 161 | 1.16 | 46.5 |
| ONG682 | ZJN296 | IgG format with CrossMab^{CH1-CL} | 1 | 1 | 161 | 0.87 | 20.1 |
| YMI345 | XVT458 | IgG with N-terminal scFv | 2 | 1 | 187 | 0.12 | 54.8 |
| CUM013 | XVT458 | IgG with C-terminal scFv, (G4S)4 linker | 2 | 1 | 188 | 0.11 | 47.3 |
| QWT744 | XVT458 | IgG with C-terminal scFv, (G4S)2 linker | 2 | 1 | 187 | 0.12 | 42.7 |

### Example 6: In vitro and in vivo cellular selectivity

In order to assess the functionality of the anti-IL-2/IL-2 fusion protein arm of the bispecific compounds, STAT5 phosphorylation was analyzed in murine splenocytes as downstream signaling of the IL-2R activation. pSTAT5 was measured in different cell populations after stimulation with Proleukin, or with one of the bispecific compounds. Mouse splenocytes were incubated with a serial dilution of bispecific compounds diluted in RPMI + 10% FBS. Starting concentration 100 nM, dilution rate 1:5 obtaining 6 total concentrations. Samples were incubated at 37 °C for 15 minutes and fixed immediately afterwards by adding same volume of cytofix buffer (BD Biosciences, Cat#554655) per sample and incubating samples for 10 minutes at 37 °C. After fixation cells were stained with BV421 rat anti-mouse CD25 (clone PC61, BD Biosciences, 0.5 *µ*L/samples), BV650 hamster anti-mouse CD3e (clone 145-2C11, BD Biosciences, 1 *µ*L/samples), BV711 mouse anti-mouse NK1.1 (clone PC136, BD Biosciences, 0.33 *µ*L/samples) for 30 minutes at room temperature. Subsequently, cells were permeabilized using Perm Buffer III (BD Biosciences, Cat#558050) incubating for 10 minutes on ice. A second staining included PE-CF594 rat anti-mouse CD4 (clone RM4-5, BD Biosciences, 0.25 *µ*L/samples), APC-780 rat anti-mouse CD8b (clone H35-17.2, eBioscience, 0.167 *µ*L/samples), AF488 rat anti-mouse FoxP3 (clone FJK-16, eBioscience, 0.5 *µ*L/samples), AF647 mouse anti-mouse pSTAT5 (clone pY694, BD Bioscience, 20 *µ*L/samples). All samples were acquired with the software SpectroFlo^{®} on the Cytek^{®} Aurora. The .fcs files were analyzed with FlowJo_v10.6.2. EC50 of %pSTAT5+ values were obtained by sigmoidal 4-PL fitting. % of pSTAT5+ cells were plotted for each cell population and each tested compound against the log concentration (M).

The obtained EC50 values are shown in Table 19. All formats, except the IgG C-terminal scFv bispecifics, showed comparable (or max. 10 fold lower) potency in inducing STAT5 phosphorylation in NK and CD8 T cells compared to Proleukin (Table 19). EC50 values for QWT744 and CUM013 could not be determined, as the %pSTAT5 positive cells with the highest concentration of compound tested (100 nM) did not reach maximal levels. All compounds showed reduced potency in inducing pSTAT5 in Treg cells compared to Proleukin, indicating that signaling through the high affinity trimeric IL-2R is hindered *in vitro* (Table 19).

**Table 19 Bispecific compounds, QTY065, and Proleukin EC50 values for pSTAT5 positive cells in parental NK, CD8, and Treg cells measured by flow cytometry in mouse splenocytes.**

| Compound ID | EC50 pSTAT5 on NK cells (nM) | EC50 pSTAT5 on CD8 cells (nM) | EC50 pSTAT5 on Treg cells (nM) |
|---|---|---|---|
| Proleukin | 2.51 | 11.92 | < 0.02 |
| QTY065 | 2.88 | 17.93 | 4.21 |
| HRL470 | 4.04 | 27.45 | 1.18 |
| VNP090 | 14.67 | 26.81 | 10.94 |
| FQQ111 | 27.34 | 34.1 | 6.55 |
| YMI345 | 23.77 | 29.6 | 13.67 |
| CUM013 | > 100.00 | > 100.00 | > 100.00 |
| QWT744 | > 100.00 | > 100.00 | > 100.00 |
| ONG682 | 14.08 | 23.87 | 12.72 |

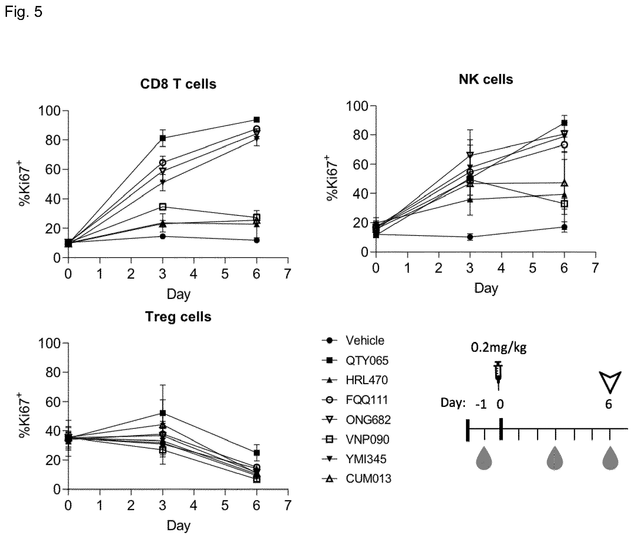

To confirm selectivity towards CD122-CD132 expressing cells *in vivo,* bispecific compounds were administered i.v. at 0.2mg/kg to wt C57BL/6 mice. Blood samples were taken before the injection and on days 3 and 6 after compound administration. Cell proliferation was analyzed by flow cytometry, detecting Ki67+ cells in CD8 T cells, NK cells and Treg cells (Fig. 5). C57BL/6 mice were injected i.v. with a single dose of 0.2 mg/kg compound. Blood samples taken before compound administration and on days 3 and 6 after compound administration were processed and analyzed by flow cytometry. Cells were first stained for CD25, CD3, NK1.1, CD4, CD8 and Zombie aqua fixable viability dye (Biolegend). After fixation and permeabilization (eBioscience) intracellular staining was performed for Ki67 and FoxP3. All compounds showed only slight increase in Ki67 expression in Treg cells on day 3, with levels decreasing on day 6 after compound administration. Proliferation (Ki67 expression) of CD8 T cells and NK cells in the blood of animals treated with bispecific compounds in the IgG format (FQQ111 and ONG682) or IgG-N-ter scFv (YIM345) format was comparable to QTY065, reaching maximal levels of Ki67+ cells (>75%) on day 6 after administration. Compounds with smaller molecular weight (Mw) and lacking the Fc domain, as HRL470 and VNP090, showed Ki67+ levels of 22-25% in CD8 T cells and 33-40% in NK cells. The bispecific format with C-terminal fusion of anti-hIL-2/IL-2 scFv on the anti-PD-1 IgG (CUM013) showed reduced potency compared to the other Fc-domain bearing compounds, with 47% Ki67+ NK cell and 25% Ki67+ CD8T cells, suggesting surprisingly, the IL-2 signaling was less potent when the cytokine was in a C-terminal linkage to a targeting antibody domain (Fig. 5).

### Example 7: In vivo anti-tumor efficacy

The B16F10 melanoma model, an aggressive, and immune checkpoint inhibitor (CPi) resistant tumor model, was selected to investigate additional protection conferred by non-blocking, PD-1-targeted moieties compared to anti-IL-2/IL-2 fusion proteins lacking PD-1 targeting. Immune cell proliferation and immunophenotyping of B16F10 subcutaneous tumors was performed in humanized PD-1 mice so that both PD-1 targeting and IL-2 activity could be assessed (C57BL/6N-Pdcd1tm1(huPDCD1-ICP11; Geno). Transgenic hPD-1 mice were injected s.c. in the right flank with 1x 10⁶ B16F10 cells. When tumors reached an average size of 70 to 100 mm³ compounds were administered i.v. at a dose of 0.2 mg/kg. Tumor volumes were measured daily with a caliper and calculated with the formula (length x width x width) 12. Tumor volume differences in % compared to vehicle at study end at day 6 after treatment onset were calculated. Tumor infiltrating cells were analyzed by flow cytometry as described above, using the antibodies shown in Table 20.

**Table 20: Antibodies used for intracellular (IC) and extracellular staining (surface) for immunophenotyping of B16F10 tumors.**

| | **Antigen** | **Label** | **Clone** | **Supplier** | **Catalog No** |
|---|---|---|---|---|---|
| Surface | CD45 | BV480 | 30-F11 | BD Biosciences | 566095 |
| | CD11b | BV605 | M1/70 | BD Biosciences | 563015 |
| | F4/80 | BV605 | T45-2342 | BD Biosciences | 743281 |
| | CD3 | BV650 | 145-2C11 | BD Biosciences | 564378 |
| | CD4 | PE-CF594 | RM4-5 | BD Biosciences | 562285 |
| | CD8 | R718 | H35-17.2 | BD Biosciences | 752290 |
| | CD25 | BB515 | PC61 | BD Biosciences | 564424 |
| | CD19 | SparkBlue | 6D5 | Biolegend | 115566 |
| | hPD1 | PE | A17188B | Biolegend | 621607 |
| | CX3CR1 | BV785 | SA011F11 | Biolegend | 149029 |
| | NK1.1 | BV711 | PK136 | BD Biosciences | 740663 |
| | CD49b | BB700 | HMα2 | BD Biosciences | 742140 |
| IC | FoxP3 | BV421 | MF23 | BD Biosciences | 562996 |
| | TCF1 | AF647 | C63D9 | Cell Signaling | 6709 |
| | Ki67 | PE-Cy7 | RMT3-23 | Biolegend | 119716 |

While administration of the untargeted anti-IL-2/IL-2 fusion protein QTY065 mainly led to increased intratumoral NK cells in the tumor of treated mice (8.2 fold compared to vehicle), all PD-1 targeted compounds drove a marked increase of CD8 T cells in B16F10 tumors. The smaller format HRL470 with double scFv fusion has the smallest effect (2.7 fold increase compared to vehicle), while the IgG and IgG-scFv fusion lead to CD8 T cell increase 4.6-8.1 fold compared to vehicle. Notably, the effect was enhanced within the target cell population, CD8+PD-1+ cells (Table 21). The C-terminal scFv compound CUM013, which did not show high potency in previous assays, showed comparable effects to the other bispecific formats on tumor infiltrating lymphocytes (TILs). Nonetheless, CUM013 had smaller effect on tumor growth inhibition compared to the other bispecifics (Table 22). Based on the results obtained, heterotetrameric IgG (CrossMab format) and IgG-N'terminal-scFv-IL-2-fusion format bispecific molecules were particularly effective *in vivo,* and the formats of VNP090 and CUM013 were not pursued further and shelved as potential backup.

**Table 21: TILs from transgenic hPD-1 mice treated with untargeted or PD-1 targeted IL-2 antibody fusion protein as fold increase cells/gram tumor over vehicle. Tumors analyzed on day 6 (n=4).**

| | Fold over vehicle cells/gram tumor | | | |
|---|---|---|---|---|
| Compound ID | Treg cells | NK cells | CD8 T cells | CD8 PD-1+ |
| QTY065 | 0.82 | 8.21 | 1.73 | 0.38 |
| HRL470 | 1.81 | 0.88 | 2.75 | 3.23 |
| VNP090 | 1.51 | 1.84 | 5.26 | 5.20 |
| FQQ111 | 1.25 | 1.12 | 4.62 | 5.52 |
| YMI345 | 0.97 | 1.78 | 5.32 | 6.36 |
| CUM013 | 0.83 | 0.80 | 8.14 | 9.83 |

**Table 22: Tumor volume difference in % compared to mice treated with vehicle on day 6, n=4 mice per group.**

| Compound ID | Tumor growth inhibition compared to vehicle (day 6) |
|---|---|
| QTY065 | -11.1% |
| HRL470 | 39.8% |
| VNP090 | 4.1% |
| FQQ111 | 46.6% |
| YMI345 | 33.0% |
| CUM013 | 8.7% |

In an additional study, tumor growth in hPD-1 transgenic mice was monitored after two administrations of bispecific compounds in comparison to vehicle, a non-blocking PD-1 antibody lacking IL-2 binding (the antibody XVT458 as human IgG1, with Fc silencing), or the untargeted bivalent antibody-IL-2 fusion QTY065. Day 0 corresponds to study start, when tumors reached 70-100mm³. On day 0, and 3, 1.25 nmoles / kg of each compound were administered i.v. tumor growth was monitored and tumor volume difference was compared to mice treated with vehicle at end of study (Table 23). On day 5 after treatment, tumors were processed and TILs were analyzed by flow cytometry with intracellular staining as shown in Table 20. The inventors determined the fold increase compared to vehicle of cells/gram tumor of Treg cells, NK cells, CD8 T cells and CD8 T-cells subfamilies including stem-like pre-exhausted CD8 T cells (CD8+PD-1+TCF1+), better effector exhausted T cells (CD8+TCF1-CX3CR1+) and terminally exhausted T cells (CD8+TCF1-CX3CR1-). The control antibody XVT458 induced only a minimal increase of the tumor cell infiltrate. The bispecific compounds induced in particular increase of CD8+PD-1+ T cells and derived subfamilies, with the heterotetrameric IgG CrossMab and IgG-scFv N-terminal format being the most efficacious compounds, with regards to target cell targeting, and anti-tumor potency (Tables 23 and 24).

**Table 23: Tumor volume difference in % compared to mice treated with vehicle on d5 (n=5-6).**

| Compound ID | Tumor growth inhibition % |
|---|---|
| QTY065 | 34.6% |
| HRL470 | 41.0% |
| FQQ111 | 70.5% |
| ONG682 | 59.3% |
| YMI345 | 54.5% |
| XVT458 | 17.4% |

**Table 24: TILs from transgenic hPD-1 mice treated with untargeted or PD-1 targeted IL-2 antibody fusion protein as fold increase cells/gram tumor over vehicle. Tumors analyzed on day 5 post initiation of treatment.**

| | Fold over vehicle cells/gram tumor | | | | | | |
|---|---|---|---|---|---|---|---|
| Compound ID | Treg cells | NK cells | CD8 T cells | CD8 PD-1+ | CD8+PD-1+ stem-like | CD8+PD-1+ better effector | CD8+PD-1 + TCF1-exhausted |
| QTY065 | 1.7 | 63.0 | 4.9 | 3.5 | 13.1 | 3.4 | 5.3 |
| HRL470 | 2.2 | 3.6 | 3.1 | 3.0 | 3.9 | 2.8 | 4.5 |
| FQQ111 | 5.3 | 15.4 | 30.7 | 31.9 | 33.1 | 21.7 | 35.2 |
| ONG682 | 5.1 | 9.4 | 28.4 | 30.1 | 20.6 | 20.7 | 32.8 |
| YMI345 | 4.1 | 14.6 | 22.1 | 48.4 | 39.0 | 30.3 | 43.3 |
| XVT458 | 2.4 | 2.6 | 3.5 | 3.6 | 1.7 | 3.1 | 4.0 |

### Example 8: Increased in vitro potency of PD-1 targeted anti-hIL-2/IL-2 fusion proteins

Select bispecific formats were then combined with well-performing non-blocking anti-hPD-1 antibodies (Table 25). Antibodies targeting irrelevant antigens MDC982 and KVC110 were used as untargeted control antibodies. The anti-hIL-2/IL-2 arm from QTY065 has a light chain from the kappa subfamily, and can be combined with a second arm being from the lambda subfamily to form a heterotetrameric compound (a bispecific antibody where each antigen binding domain has a different specificity), without need for genetic engineering strategies such as CrossMab to ensure correct light chain pairing. Bispecifics in the heterotetrameric IgG kappa/lambda (Figure 4F) format were designed combining the IL-2-anti-IL-2 fusion portion of QTY065 with two representative non-blocking, high affinity PD-1 antibodies 21A08Ap1 and 21A08Ap2 (both having a lambda light chain). Constructs encoding the sequences disclosed in Table 25 were transfected into Expi293 cells and produced and purified as described above. Knob in hole mutations were Y407T on HC1 and T366Y on HC2, and Fc silencing mutations L234A, L235A, P329A were incorporated.

**Table 25: Combinations of QTY065 VH, VL(IL-2), and a second anti-hPD1 (or control) antibody heterodimer in 3 different bispecific antibody formats.**

| **Anti-PD-1 Antibody (light chain)** | **Bispecific antibody formats combined to QTY065 (kappa) (SEQ ID NO of polypeptide components)** | | |
|---|---|---|---|
| | **IgG CrossMab** | **IgG-scFv N-terminal** | **IgG kappa/lambda** |
| 21A08A p1 (lambda) | YPW986 (088, 089, 090, 052) | LTJ498 (094, 095, 096) | XWY176 (094, 095, 100, 052) |
| 21A08A p2 (lambda) | PXU588 (092, 093, 090, 052) | JLI141 (097, 098, 099) | GQM289 (097, 098, 100,052) |
| XVT458-z2-m3 (kappa) | RIB426 (101, 102, 090, 052) | TMU471 (105, 106, 107) | - |
| XVT458-z2-m6 (kappa) | QAB373 (103, 104,090,052) | MDS446 (108, 109, 110) | - |

| **Control Antibody (light chain)** | **IgG CrossMab** | **IgG-scFv N-terminal** | **IgG kappa/lambda** |
|---|---|---|---|
| KVC110 (kappa) | TSQ225 | LNX431 | - |
| MDC982 (lambda) | - | - | UVW948 |

The bispecific compounds were tested for potency in inducing STAT5 phosphorylation in PD-1+ Jurkat cells, expressing the IL-2R CD122-CD132. Jurkat-PD1+ CD122+ cells were activated with a serial dilution of bispecific compounds for 15 minutes at 37°C and fixed by adding an equal volume of Cytofix buffer (BD Biosciences) for 10 minutes at 37°C. For the staining of intracellular antigens, cells were permeabilized with ice cold Perm buffer III (BD Biosciences) for 15 minutes on ice. Phosphorylated STAT5 was stained using an anti-pSTAT5 pY694 antibody (clone 47/Stat5, BD Biosciences). Flow cytometry was performed as previously described. EC50 values were calculated using the formula Y=Bottom + (X^Hillslope)*(Top-Bottom)/(X^HillSlope + EC50^HillSlope) in Graphpad Prism V9.3.1 ([Agonist] vs. response - Variable slope (four parameters)). The PD-1 targeted bispecific compounds, except YPW986 and PXU588, showed increased potency and lower EC50 values compared to the bivalent QTY065 and the untargeted compound TSQ225 (Table 26). The binding ELISA assay suggested YPW986 and PXU588 (i.e. heterotetrameric IgG antibodies in a CrossMab bispecific format, with one arm derived from QTY065 and one either from 2108Ap1 or 21A08Ap2) lost their binding affinity to PD-1, in contrast to the heterotetrameric kappa/lambda IgG formats using the same non-blocking anti-PD-1 clones XWY176 and GQM289, which retained their ability to stimulate the dimeric IL-2 receptor. In general, PD-1 anchoring to the cells increased the signaling potency of the IL-2 on the bispecific compounds. The heterotetrameric Crossmab bispecific IgG FQQ111 with non-affinity matured anti-PD-1 arm (XVT458) induced a lower increase in potency by targeting to PD-1, indicating that higher affinity binding is desirable.

**Table 26: EC50 values for STATS phosphorylation on Jurkat-PD-1+ CD122+ cells.**

| Compound | EC50 value for pSTAT5 induction (nM) |
|---|---|
| MDS446 | 0.06 |
| TMU471 | 0.06 |
| QAB373 | 0.07 |
| RIB426 | 0.12 |
| JLI141 | 0.20 |
| GQM289 | 0.22 |
| LTJ498 | 0.24 |
| XWY176 | 0.27 |
| FQQ111 | 0.62 |
| QTY065 | 1.07 |
| TSQ225 | 5.16 |
| PXU588 | 5.37 |
| YPW986 | 43.9 |

A further functional effect of a fusion protein according to the invention, is decrease of cell surface PD-1 upon binding to the IL-2R. Signaling through the IL-2R leads to internalization of the dimeric or trimeric receptor complex (Robb RJ. Et al. J Exp. Med. (1987) doi: 10.1084/jem. 165.4.1201). Reduction of cell surface hPD-1 and CD122 by immunoconjugates of the present invention was tested by incubating the bispecifics at 3 different concentrations with stimulated PBMCs (i.e., PD-1+) for 16 hours. As controls, the bivalent anti-hlL-2/IL-2 compound QTY065 and the untargeted IL-2 compound as IgG CrossMab were used. Buffy coats or whole blood from healthy volunteers were received from the Blutspendezentrum SRK beider Basel or Aarau in compliance with the Swiss Human Research Act (HRA; May 2021) and other applicable ethics regulations by the Swiss ethics committee. PBMCs were isolated by density gradient centrifugation using Ficoll Plaque Plus (GE Healthcare). Frozen PBMCs were defrosted activated for 3 days with plate coated anti-CD3 (clone OKT3, BioLegend) and soluble anti CD28 (clone CD28.2, BioLegend). After activation PBMCs were incubated with bispecific compounds as indicated, overnight at 37°C. After incubation, cells were fixed immediately by adding an equal volume of Cytofix buffer (BD Biosciences) for 10 minutes at 37°C. Subsequently, surface markers were stained using antibodies listed in Table 27. Cells were acquired by flow cytometry as described above.

**Table 27: Antibodies used for extracellular staining for cell surface expression experiments.**

| **Antigen** | **Label** | **Clone** | **Supplier** | **Catalog No** |
|---|---|---|---|---|
| CD4 | PE-CF594 | SK3 | BD Biosciences | 566914 |
| CD8 | BV605 | SK1 | BD Biosciences | 564116 |
| CD122 | PE Cy7 | CF1 | BeckmanCoulter | A53365 |
| PD-1 | BB700 | EH12.1 | BD Biosciences | 746185 |
| CD3 | BV786 | SK7 | BD Biosciences | 563800 |
| CD132 | APC | TUGh4 | Biolegend | 338608 |
| CD25 | BV421 | 2A3 | BD Biosciences | 564033 |
| CD4 | PE-CF594 | SK3 | BD Biosciences | 566914 |

The difference in MFI of detected surface CD122 and PD-1 was calculated as % decrease compared to PBMC that were incubated only in medium. The control compounds induce decrease surface CD122, but leave PD-1 levels unchanged. For the PD-1 targeting compounds, both CD122 and PD-1 levels decreased. This may indicate that binding to the IL-2R is required for reduction of cell surface PD-1 (Table 28).

**Table 28: Percentage decrease of CD122 and PD-1 MFI on CD8 T cells after incubation with control compounds or bispecific compounds compared to CD8 T cells incubated with medium alone (n= 2 donors).**

| | % MFI decrease | | | | | |
|---|---|---|---|---|---|---|
| | CD122 | | | PD-1 | | |
| Compound | 25nM | 5nM | 1 nM | 25nM | 5nM | 1 nM |
| QTY065 control | 76.6 | 67.0 | 41.6 | - | - | - |
| TSQ225 control | 78.0 | 61.7 | 40.7 | - | - | - |
| LTJ498 | 59.1 | 36.2 | 7.8 | 46.4 | 46.7 | 41.8 |
| XWY176 | 62.9 | 40.1 | 9.4 | 52.4 | 52.7 | 42.0 |
| JLI141 | 60.7 | 35.3 | 5.7 | 38.6 | 45.0 | 38.0 |
| GQM289 | 57.4 | 39.9 | 9.7 | 46.4 | 44.8 | 25.7 |
| TMU471 | 59.8 | 43.4 | 24.7 | 34.6 | 46.4 | 45.0 |
| RIB426 | 56.9 | 41.3 | 15.4 | 43.3 | 46.7 | 33.2 |
| MDS446 | 76.5 | 59.5 | 32.2 | 29.1 | 37.3 | 31.1 |
| QAB373 | 63.3 | 41.6 | 24.4 | 33.0 | 30.6 | 27.3 |

### Example 9: Improved PD-1 binders: in vivo anti-tumor efficacy and s.c. tumor immunophenotyping

The improved bispecific compounds of Table 25 were tested on hPD-1 transgenic mice bearing s.c. B16F10 tumors. Transgenic hPD-1 mice (C57BL/6N-Pdcd1tm1(huPDCD1-ICP11)Geno) were injected s.c. in the right flank with 1x 10⁶ B16F10 cells. When tumors reached an average size of 70 to 100 mm³ (day 0), compounds were administered i.v. at 1.25 nmoles/kg. A second administration was given on day 3. Mice were sacrificed on day 5 after study start and tumors were excised. Tumors were processed through a GentleMACS Octo Dissociator (Milteny) and cells were stained with NIR live dead stain (ThermoFisher, L10119). Cells were incubated in Fc Block (TruStain FcX^{™}, Biolegend, 101320) 10 min before adding the surface stain (see Table 20). Cells were fixed and permeabilized with FoxP3 Staining Buffer Set (ebioscience, #00-5523-00) before intracellular staining as per Table 20. All PD-1 targeted anti-IL-2/IL-2 bispecific antibodies markedly increase the intratumoral CD8 T cell numbers, in particular PD-1+ stem-like T cells and the derivatives compared to untargeted compounds or vehicle (Table 29). CD8 T cells / Treg ratio was increased in the tumors of mice treated with bispecific compounds compared to vehicle or untargeted bispecifics.

**Table 29. TILs from transgenic hPD-1 mice treated with untargeted or PD-1 targeted IL-2 antibody fusion protein as fold increase cells/gram tumor over vehicle. Last column reports CD8 to Treg cells ratios. Tumors analyzed on day 5.**

| | Fold over vehicle cells/gram tumor | | | | | | | CD8/Treg ratio of cells /gram tumor |
|---|---|---|---|---|---|---|---|---|
| Compound | Treg cells | NK cells | CD8 T cells | CD8 PD-1 + | CD8+PD-1+ stem-like | CD8+PD-1+ better effector | CD8+PD-1+TCF1-exhausted | |
| Vehicle | - | - | - | - | - | - | - | 9.3 |
| RIB426 | 8.5 | 15.8 | 25.6 | 28.1 | 22.8 | 25.5 | 40.6 | 27.9 |
| TMU471 | 20.5 | 37.0 | 107.1 | 122.7 | 121.5 | 112.7 | 156.3 | 48.4 |
| QAB373 | 13.7 | 28.0 | 79.5 | 90.2 | 72.3 | 84.0 | 122.9 | 53.6 |
| MDS446 | 10.1 | 34.6 | 105.1 | 121.2 | 75.6 | 116.0 | 167.6 | 96.2 |
| XWY176 | 6.7 | 18.1 | 41.4 | 47.7 | 38.8 | 45.7 | 59.7 | 57.2 |
| LTJ498 | 12.4 | 29.0 | 69.1 | 81.0 | 51.1 | 78.1 | 110.5 | 51.6 |
| GQM289 | 8.0 | 18.8 | 27.1 | 29.6 | 24.4 | 26.6 | 42.6 | 31.4 |
| JLI141 | 2.7 | 8.2 | 29.5 | 35.6 | 16.8 | 33.1 | 56.2 | 100.4 |
| TSQ225 | 2.2 | 12.5 | 3.3 | 1.2 | 1.4 | 1.1 | 1.3 | 14.0 |
| LNX431 | 2.8 | 18.5 | 3.6 | 1.7 | 2.0 | 1.6 | 1.7 | 12.1 |

In an efficacy study, transgenic hPD-1 mice (C57BL/6N-Pdcd1tm1(huPDCD1-ICP1 1)Geno) were injected s.c. in the right flank with 1x 10⁶ B16F10 cells. When tumors reached an average size of 70 to 100 mm³ (day 0), compounds were administered i.v. at 1.25 nmoles/kg. Additional administrations were given on day 3 and 7. Tumors were measured daily and volume was calculated with the formula (length x width x width) 12. Tumor volume difference between mice treated with vehicle or the respective compound n reported as % decrease compared to vehicle. All PD-1 targeted IL-2 fusion protein compounds effectively reduced tumor volume (Table 30).

**Table 30. Tumor volume difference in % to vehicle on day 7.**

| Compound | **% TGI vs vehicle** |
|---|---|
| Vehicle | - |
| TMU471 | 59 |
| QAB373 | 58 |
| MDS446 | 80 |
| XWY176 | 69 |
| LTJ498 | 62 |
| GQM289 | 64 |
| JLI141 | 80 |
| RIB426 | 65 |

### Example 10: Stress study on anti-PD-1 , anti-IL-2/IL-2 bispecific antibodies

Heterotetrameric bispecific antibodies XWY176, TMU471, QAB373 and MDS446 were produced with improved knob-in-hole mutations (S354C, T366W / S354C, T366S, L368A, Y407V) and Fc silencing (Table 31).

**Table 31. Bispecific compounds with optimized knob-in hole mutations and Fc silencing.**

| **Bispecific antibody name combined to QTY065** | **Sequence ID** |
|---|---|
| NZA596 | 111, 095, 112, 052 |
| KTX917 | 113, 106, 114 |
| CIT348 | 115, 109, 116 |
| BGY642 | 117, 104, 112, 052 |

The four bispecific antibodies from Table 31 were exposed to thermal, pH, oxidation, freeze-thaw stress conditions. After that the compounds were tested on SEC-HPLC, iCEIF and functional ELISA Posttranslational modifications were analyzed by Mass spectrometry. The bispecific antibodies XWY176, TMU471, QAB373 and MDS446 were exposed to different stress conditions: i) incubation at 40°C for 1 or 2 weeks, ii) 3 or 4 freeze-thaw cycles, iii) in 0.1% (v/v) H₂O₂ for 4h or 24h, iv) low pH 3.5 for 24 or 48h, v) high pH 9.0 for 24 or 48h. To assess protein changes after the stress conditions the compounds were tested by SEC-HPLC, iCIEF, LC-MS. The functionality of the bispecific antibody was assessed with a sandwich ELISA that relies on its binding to the target antigen (hPD-1) and the integrity of the fused IL-2 via a secondary antibody (anti-IL-2 clone 5344). 60 nM of hPD-1 (ECD-His, produced in house) was coated on a Maxisorp plate (Nunc) overnight at 4°C and blocked with 5% BSA in PBS. Bispecific antibodies were added in a serial dilution in assay buffer and detected with biotinylated-5344 and Streptavidin HRP (BD Pharmingen, 554066). Absorption end signal after adding TMB was read with a plate reader (Spectramax ID3) at 450 nm. EC50 values were determined by blotting Absorbance against concentration (Graphpad Prism, sigmoidal curve fit, 4 PL, X-axis is log). Change of EC50 values for the unstressed sample (T0) was compared to the stressed samples.

All constructs showed high potential for further development based on the criteria assessed in this Example.

### Example 11: Mass spectrometry showing correct light chain pairing

To test correct light chain pairing of the purified bispecific constructs in the IgG CrossMab or IgG kappa/lambda formats digestion by IgdE enzyme was performed and the Fab fragments were analyzed by Mass spectrometry. One single peak was visible for each of the Fabs being assayed, corresponding to the theoretical molecular weight of correctly assembled light chain and heavy chain. No incorrect light chain pairing was detected.

### Example 12: Cis-signaling in combination with PD-1 blocking antibodies

An assay was established to assess whether an exemplary bispecific construct as disclosed herein delivers the IL-2 to CD122-CD132 on the PD-1 expressing cell bound by its anti-PD-1 arm (cis-signaling) or signals to a neighboring cell (trans). Jurkat-PD-1+ CD122+ cells were either labeled with CFSE (Invitrogen, C34557) or CTV (Invitrogen C34554). CFSE labeled cells were then exposed to 700 nm of the non-competing parent antibody to PD-1, Pembrolizumab or Nivolumab, to block PD-1 epitopes for 30 minutes at room temperature. After two washes, both CTV and CFSE labeled cells were mixed at a 1:1 ratio and activated with the bispecific immunoconjugate (1 nM) for 15 minutes at 37°C. After the stimulation period, cells were fixed immediately by adding an equal volume of Cytofix buffer (BD Biosciences 554655) for 10 minutes at 37°C. For the staining of intracellular antigens, cells were permeabilized with ice cold Perm buffer III (BD Biosciences 558050) for 15 minutes on ice. Phosphorylated STAT5 was stained using an anti-pSTAT5 pY694 antibody (clone 47/Stat5, BD Biosciences). Induction of STAT5 phosphorylation through the immunoconjugate in these cells was analyzed. The potency of the immunoconjugate is markedly reduced on cells that were pre-incubated with the parent antibody. This interference is not observed on cells were that were pre-exposed to Pembrolizumab or Nivolumab (Table 36). Furthermore, the potency of the immunoconjugate on cells that had not been exposed to any of the PD-1 binding antibodies before was the same for all samples (co-incubated with CFSE+ pre-blocked cells), indicating that the immunoconjugate signals in a cis manner, on the same cells where PD-1 binding occurs (Table 36).

**Table 36. Percentage of pSTAT5 compared to non-blocked cells after stimulation with 1 nM of an exemplary immunoconjugate:**

| | % pSTAT5 | |
|---|---|---|
| PD1 binder | CFSE labeled cells | CTV labeled cells |
| Non blocked | 100.00 | 100.00 |
| parent AB | 4.81 | 89.80 |
| Pembrolizumab | 82.59 | 82.56 |
| Nivolumab | 81.39 | 82.44 |

### Example 13: Anti-tumor efficacy of bispecific immunoconjugate in combination with Pembrolizumab and Nivolumab in two mouse models of cancer

The efficacy of an exemplary bispecific immunoconjugate (IC) disclosed in this application was tested as monotherapy or in combination with commercially available PD-1 blocking agents Pembrolizumab and Nivolumab. hPD-1 transgenic C57BL/6 mice were injected with s.c. B16F10 melanoma cells or MC38 colorectal tumor cells. When tumors reached an average size of 70-100mm³ mice were randomized and treatment was started as described in Table 37. Mice were administered on days 0 (day of randomization) and day 3. Pembrolizumab or Nivolumab were administered also on day 7. Tumor growth inhibition was calculated on day 13 for MC38 tumor bearing mice and on day 14 for B16F10 tumor bearing mice compared to tumor volumes of mice treated with Vehicle. In both tumor models the bispecific immunoconjugate combined to either of the checkpoint inhibitors induced marked tumor growth retardation even at low doses, compared to vehicle. Combination treatments led to stronger tumor growth inhibition compared to any of the single agent treatments.

**Table 37. Treatment schedules and doses for immunconjugate (I.C) in vivo efficacy study in hPD-1 transgenic mice (each group n=9) bearing B16F10 or MC38 tumors. Tumor growth inhibition was calculated as percentage volume decrease compared to mean volume of Vehicle treated group. * Dose 1: MC38 model: 0.1mg/kg, B16F10 model: 0.2mg/kg ** Dose 2: MC38 model: 0.2mg/kg, B16F10 model: 0.4mg/kg.**

| Treatment group | Compound 1 Days 0, 3 (i.v.) | Compound 2 Days 0, 3, 7 (i.p.) | Tumor growth inhibition on Day 9 in MC38 model | Tumor growth inhibition on Day 14 in B16F10 model |
|---|---|---|---|---|
| Vehicle | PBS | PBS | - | - |
| Pembrolizumab | PBS | Pembrolizumab 10mg/kg | 41.6% | 37.3 % |
| Nivolumab | PBS | Nivolumab 10mg/kg | 41.5% | 32.4 % |
| I.C dose 1* | I.C | PBS | 35.4% | 52.4 % |
| I.C dose 2** | I.C | PBS | 57.2% | 47.4 % |
| I.C dose 1* + Pembrolizumab | I.C | Pembrolizumab 10mg/kg | 65.7% | 68.8 % |
| I.C dose 2* + Pembrolizumab | I.C | Pembrolizumab 10mg/kg | 74.2% | 72.2 % |
| I.C dose 1* + Nivolumab | I.C | Nivolumab 10mg/kg | 76.7% | 74.7 % |
| I.C dose 2* + Nivolumab | I.C | Nivolumab 10mg/kg | 81.8% | 76.5 % |

## Claims

1. An immunoglobulin variable domain capable of binding to PD-1 comprising
- an antibody heavy chain variable domain polypeptide (PD1-VH), and
- an antibody light chain variable domain polypeptide (PD1-VL),
**characterized in that**
- the PD1-VH comprises an HCDR1 having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120); and
- the PD1-VL comprises an LCDR1 having the sequence SGX¹SSNIGS(XX)²VF (SEQ ID NO 121), an LCDR2 having the sequence SNNQRPS (SEQ ID NO 122), and an LCDR3 having the sequence AAWDDSLSIWVF (SEQ ID NO 123);
and wherein X¹ is N, S, Q, or A, and (XX)² is NS, QS, SS, or NA.

2. The immunoglobulin variable domain according to claim 1, wherein binding of an antibody comprising the immunoglobulin variable domain as specified in claim 1 to PD-1 is no more than 20% reduced in the presence of a 100-fold molar excess of a PD-1specific antibody selected from the group of pembrolizumab and nivolumab.

3. The immunoglobulin variable domain according to claim 1 or 2, wherein the PD1-VL comprises an LCDR1 having the sequence SGASSNIGS**QS**VF (SEQ ID NO 124), SGASSNIGS**SS**VF (SEQ ID NO 125), or SGASSNIGS**NA**VF (SEQ ID NO 126);
particularly wherein the PD1-VH comprises, or consists of a polypeptide at least (≥) 95%, ≥ 98%, ≥ 99% similar to SEQ ID NO 085, and the PD1-VL comprises, or consists of a polypeptide ≥ 95%, ≥98 %, ≥ 99% similar to SEQ ID NO 086, SEQ ID NO 087, or SEQ ID NO 091;
more particularly wherein the PD1-VH comprises, or consists of a polypeptide having the sequence SEQ ID NO 085 and the PD1-VL comprises, or consists of a polypeptide having the sequence SEQ ID NO 086, SEQ ID NO 087, or SEQ ID NO 091.

4. The immunoglobulin variable domain according to any one of the claims 1 to 3, wherein the affinity constant (K_{D}) for PD-1 of an antibody **characterized by** said immunoglobulin variable domain is in the range of 1.0x10⁻⁹ to 1.5x10⁻¹¹ mol/L, particularly 1.0x10⁻¹⁰ to 1.5x10⁻¹¹ mol/, more particularly 5.0x10⁻¹⁰ to 1.5x10⁻¹¹ mol/ as measured by SPR.

5. An immunoconjugate comprising an immunoglobulin variable domain capable of binding PD-1 as specified in any one of the claims 1 to 4.

6. The immunoconjugate according to claim 5, further comprising an immune-active polypeptide ligand capable of binding to a cell surface molecule expressed by T cells or natural killer cells.

7. The immunoconjugate according to claim 6, wherein the immune-active polypeptide ligand comprises an interleukin, and/or an immunoglobulin variable domain reactive to an interleukin.

8. The immunoconjugate according to claim 6 or 7, wherein the immune-active polypeptide ligand comprises, or consists of an interleukin and an immunoglobulin variable domain reactive to said interleukin;
wherein optionally one or two peptide linkers link the interleukin to the immunoglobulin variable domain reactive to said interleukin.

9. The immunoconjugate according to any one of claims 5 to 8, wherein the immunoconjugate comprises a fragment crystallizable (Fc) immunoglobulin domain, particularly an IgG Fc domain, more particularly an IgG Fc **characterized by** the presence of one or more modifications to constant regions of the heavy chains to enhance correct heavy chain pairing, particularly a set of knob and hole modifications selected from:
- knob: S354C, T366W and hole: Y349C, T366S, L368A, Y407V;
- knob: T366Y, and hole Y407T;
- knob: Y349C, T366W, and hole: S354C, T366S, L368A, Y407V; or
- knob: T366W, and Hole: Y407A, T366S, L368A.

10. The immunoconjugate according to any one of the claims 6 to 9, wherein the immunoconjugate is a heterotetrameric IgG comprising
- a first antibody heavy and light chain heterodimer comprising the immunoglobulin variable domain capable of binding PD-1 as specified in any in of the claims 1 to 4 **characterized in that** the light chain is a lambda light chain;
and wherein the immune-active polypeptide ligand comprises, or consists of
- a second antibody heavy and light chain heterodimer comprising an immunoglobulin variable domain reactive to a cell surface molecule expressed by immune cells, **characterized in that** the light chain is a kappa light chain.

11. The immunoconjugate according to any one of the claims 6 to 9, wherein the immunoconjugate is an immunoglobulin single chain variable fragment (scFv) format comprising:
- an anti-PD1 antibody comprising a first and a second antibody heavy chain and light chain heterodimer each comprising the anti-PD-1 immunoglobulin variable domain as specified in any in of the claims 1 to 4; and
wherein the immune-active polypeptide ligand comprises, or consists of
- an interleukin, and an immunoglobulin scFv domain reactive to said interleukin, wherein the interleukin is covalently linked to the immunoglobulin scFv domain reactive to said interleukin to provide a single contiguous recombinant polypeptide.

12. The immunoconjugate according to any one of the claims 7 to 11, wherein the interleukin is an IL-2 polypeptide, or a circularly permuted IL-2 (IL2CP) polypeptide.

13. An immunoconjugate according to any one of the claims 6 to 12, wherein the immune active polypeptide ligand comprises a polypeptide having the sequence of SEQ ID NO 167 and a polypeptide having the sequence of SEQ ID NO 043, particularly, wherein the immunoconjugate comprises, or consists of the polypeptides having the sequences:
a. SEQ ID NO 111, SEQ ID NO 095, SEQ ID NO 112, and SEQ ID NO 052;
b. SEQ ID NO 094, SEQ ID NO 095, SEQ ID NO 100, and SEQ ID NO 052;
c. SEQ ID NO 097, SEQ ID NO 098, SEQ ID NO 100, and SEQ ID NO 052;
d. SEQ ID NO 094, SEQ ID NO 095, and SEQ ID NO 96; or
e. SEQ ID NO 097, SEQ ID NO 098, and SEQ ID NO 99.

14. An isolated nucleic acid encoding the immunoconjugate according to any one of claims 1 to 13; particularly wherein the isolated nucleic acid is comprised in a mammalian expression vector under control of a promoter operable in a mammalian cell.

15. The immunoconjugate as specified in any one of the claims 1 to 13, for use in treating a cancer patient receiving concurrent treatment with an anti-PD-1 antagonist antibody, particularly an anti-PD-1 antagonist antibody selected from the list consisting of nivolumab, pembrolizumab, dostarlimab, sintilimab, tislelizumab, cemiplimab, cetrelimab, sasanlimab.
